# EUROPEAN PATENT SPECIFICATION

(11) **EP 2 399 925 B1**
(45) Date of publication and mention of the grant of the patent: **19.08.2015**
(21) Application number: 10750877.2
(22) Date of filing: 10.03.2010
(51) Int. Cl.: C07K 1/13, C07D 311/82, C07D 405/12, C07D 405/14, C07K 14/195, C07K 19/00, C09K 11/06, C12N 15/09

(54) **METHOD FOR FLUORESCENTLY LABELING PROTEIN**
VERFAHREN ZUR FLUORESZENZMARKIERUNG VON PROTEINEN
PROCÉDÉ DE MARQUAGE PAR FLUORESCENCE D'UNE PROTÉINE

(30) Priority: 10.03.2009 JP 2009056305
(43) Date of publication of application: 28.12.2011
(73) Proprietor: Osaka University, Suita-shi Osaka 565-0871 (JP)
(72) Inventor: KIKUCHI, Kazuya, Suita-shi Osaka 565-0871 (JP); HORI, Yuichirou, Suita-shi Osaka 565-0871 (JP); UENO, Hideki, Suita-shi Osaka 565-0871 (JP)
(74) Representative: Owen, Deborah Jane
(86) International application number: PCT/JP2010/054024
(87) International publication number: WO 2010/104124

(56) References cited:
- US-A1- 2004 029 230
- GRONEMEYER, THOMAS ET AL.: 'Adding value to fusion proteins through covalent labelling.' CURRENT OPINION IN BIOTECHNOLOGY vol. 16, no. 4, 2005, pages 453 - 458, XP27676732
- IMAMOTO YASUSHI ET AL.: 'Structure and photoreaction of photoactive yellow protein, a structural prototype of the PAS domain superfamily.' PHOTOCHEMISTRY AND PHOTOBIOLOGY vol. 83, no. 1, 2007, pages 40 - 49, XP008153194
- VAN DER HORST ET AL.: 'Binding, tuning and mechanical function of the 4-hydroxy-cinnamic acid chromophore in photoactive yellow protein.' PHOTOCHEMICAL & PHOTOBIOLOGICAL SCIENCES vol. 6, no. 5, 2007, pages 571 - 579, XP008153195
- BRUDLER, RONALD ET AL.: 'Coupling of hydrogen bonding to chromophore conformation and function in photoactive yellow protein.' BIOCHEMISTRY vol. 39, no. 44, 2000, pages 13478 - 13486, XP008153196
- TAKASUKA HIDEO ET AL.: 'Intramolecular fluorescence resonance energy transfer system with coumarin donor included in beta- cyclodextrin.' ANALYTICAL CHEMISTRY vol. 73, no. 5, 2001, pages 939 - 942, XP008153198
- CHEN, LEI ET AL.: 'Site-specific fluorescent labeling approaches for naringenin, an essential flavonone in plant nitrogen-fixation signaling pathways.' THE JOURNAL OF ORGANIC CHEMISTRY vol. 73, no. 21, 2008, pages 8279 - 8285, XP008153199
- HORI YUICHIRO ET AL.: 'Photoactive yellow protein-based protein labeling system with turn-on fluorescence intensity.' JOURNAL OF THE AMERICAN CHEMICAL SOCIETY vol. 131, no. 46, November 2009, pages 16610 - 16611, XP008153200

### SEQUENCE LISTING

<110> Osaka University
<120> A Method For Fluorescent Labeling Of Protein
<130> H3609
<150> JP2009-056305
   <151> 2009-03-10
<160> 31
<170> PatentIn version 3.5
<210> 1
   <211> 125
   <212> PRT
   <213> Halorhodospira halophila
<400> 1
   Met Glu His Val Ala Phe Gly Ser Glu Asp Ile Glu Asn Thr Leu Ala
   1 5 10 15
   Lys Met Asp Asp Gly Gln Leu Asp Gly Leu Ala Phe Gly Ala Ile Gln
   20 25 30
   Leu Asp Gly Asp Gly Asn Ile Leu Gln Tyr Asn Ala Ala Glu Gly Asp
   35 40 45
   Ile Thr Gly Arg Asp Pro Lys Gln Val Ile Gly Lys Asn Phe Phe Lys
   50 55 60
   Asp Val Ala Pro Cys Thr Asp Ser Pro Glu Phe Tyr Gly Lys Phe Lys
   65 70 75 80
   Glu Gly Val Ala Ser Gly Asn Leu Asn Thr Met Phe Glu Tyr Thr Phe
   85 90 95
   Asp Tyr Gln Met Thr Pro Thr Lys Val Lys Val His Met Lys Lys Ala
   100 105 110
   Leu Ser Gly Asp Ser Tyr Trp Val Phe Val Lys Arg Val
   115 120 125
<210> 2
   <211> 124
   <212> PRT
   <213> Rhodobacter capsulatus
<400> 2
   Met Glu Ile Ile Pro Phe Gly Thr Asn Asp Ile Asp Asn Ile Leu Ala
   1 5 10 15
   Arg Glu Pro Ala Arg Ala Glu Ser Leu Pro Phe Gly Ala Val Leu Leu
                   20                  25                  30
   Asp Arg Met Gly Arg Ile Ala Lys Tyr Asn Lys Ala Glu Gly Leu Ile
           35                  40                  45
   Ala Gly Arg Asp Pro Ser Thr Val Ile Gly Arg Asp Phe Phe Asn Glu
       50                  55                  60
   Ile Ala Pro Cys Ala Lys Gly Lys Arg Phe His Gly Glu Phe Leu Lys
   65                  70                  75                  80
   Phe Asn Arg Thr Gly Gln Ala Asn Val Met Leu Asp Tyr Lys Phe Asn
                   85                  90                  95
   Tyr Lys Gly Ala Glu Val Ala Val Lys Ile His Leu Lys Ser Gln Pro
               100                 105                 110
   Asp Gly Gln Phe Cys Trp Leu Phe Val Lys Arg Ala
           115                 120
<210> 3
   <211> 124
   <212> PRT
   <213> Rhodobacter sphaeroides
<400> 3
   Met Glu Ile Ile Pro Phe Gly Ser Ala Asp Leu Asp Asn Ile Leu Ala
   1               5                   10                  15
   Arg Glu Pro Gln Arg Ala Glu Tyr Leu Pro Phe Gly Ala Val Leu Leu
               20                  25                  30
   Asp Arg Thr Gly Thr Ile Leu Lys Tyr Asn Arg Ala Glu Gly Gly Ile
           35                  40                  45
   Ala Asn Arg Asn Pro Ala Asp Val Ile Gly Lys Asn Phe Phe Asn Glu
       50                  55                  60
   Ile Ala Pro Cys Ala Lys Gly Lys Arg Phe His Gly Glu Phe Leu Arg
   65                  70                  75                  80
   Phe His Gln Thr Gly Gln Val Asn Val Met Phe Asp Tyr Lys Phe Ala
                   85                  90                  95
   Tyr Lys Gly Ala Asn Val Gly Val Lys Ile His Met Lys Ser Gln Pro
               100                 105                 110
   Asp Gly Gln Ser Cys Trp Leu Phe Val Lys Arg Val
           115                 120
<210> 4
   <211> 125
   <212> PRT
   <213> Rhodothalassium salexigens
<400> 4
   Met Glu Met Ile Lys Phe Gly Gln Asp Asp Ile Glu Asn Ala Met Ala
   1               5                   10                  15
   Asp Met Gly Asp Ala Gln Ile Asp Asp Leu Ala Phe Gly Ala Ile Gln
               20                  25                  30
   Leu Asp Glu Thr Gly Thr Ile Leu Ala Tyr Asn Ala Ala Glu Gly Glu
           35                  40                  45
   Leu Thr Gly Arg Ser Pro Gln Asp Val Ile Gly Lys Asn Phe Phe Lys
       50                  55                  60
   Asp Ile Ala Pro Cys Thr Asp Thr Glu Glu Phe Gly Gly Arg Phe Arg
   65                  70                  75                  80
   Glu Gly Val Ala Asn Gly Asp Leu Asn Ala Met Phe Glu Tyr Val Phe
                   85                  90                  95
   Asp Tyr Gln Met Gln Pro Thr Lys Val Lys Val His Met Lys Arg Ala
               100                 105                 110
   Ile Thr Gly Asp Ser Tyr Trp Ile Phe Val Lys Arg Val
           115                 120                 125
<210> 5
   <211> 135
   <212> PRT
   <213> Stigmatella aurantiaca
<400> 5
   Met Arg His Gly Ile Leu Glu Ala Glu Ser Leu Thr Glu Asp Arg Leu
   1               5                   10                  15
   Gly Gln Leu Ser Pro Glu Glu Phe Asp Ala Leu Pro Phe Gly Ala Ile
               20                  25                  30
   Lys Leu Asp Ala Glu Gly Arg Val Leu Ile Tyr Asn Ala Ala Glu Ser
           35                  40                  45
   Ala Phe Ser Arg Arg Lys Pro Val Ser Val Leu Gly Arg Arg Phe Phe
       50                  55                  60
   Glu Glu Val Ala Pro Cys Thr Asn Val Ala Ser Phe Arg Gly Arg Phe
   65                  70                  75                  80
   Asp Thr Leu Val Glu Arg Gly His Gly Thr Glu Ser Phe Asp Phe Gln
                   85                  90                  95
   Phe Arg Phe Arg Trp Gly Thr Arg Asn Val Arg Ile Arg Leu Met Val
               100                 105                 110
   Leu Gly Asp Gly Ser Arg Trp Val Phe Val Thr Ala Val Leu Thr Ala
           115                 120                 125
   Leu Ile Pro Leu Gly Glu Gly
       130                 135
<210> 6
   <211> 131
   <212> PRT
   <213> Sorangium cellulosum
<400> 6
   Met Gly Ser Glu Glu Arg Ser Thr Ala Gly Glu Phe Glu Phe Asp Ile
   1               5                   10                  15
   Gly Val Phe Asn Leu Asp Glu Arg Gly Leu Asp Ala Gln Pro Phe Gly
               20                  25                  30
   Ile Ile Arg Leu Asp Arg Glu Gly Thr Val Leu Ser Tyr Asn Leu Tyr
           35                  40                  45
   Glu Glu Arg Gln Ala Arg Arg Asn Arg Gln Asp Val Ile Gly Lys Asn
       50                  55                  60
   Phe Phe Thr Asp Ile Ala Pro Cys Ser Arg Val Lys Ala Phe His Gly
   65                  70                  75                  80
   Arg Phe Leu Ala Gly Val Glu Gln Arg Glu Leu Lys Ala Thr Phe Gly
                   85                  90                  95
   Phe Val Phe His Phe Pro His Lys Thr Arg His Val Asp Val Ser Leu
               100                 105                 110
   Phe Tyr Lys Ala Ala Ala Arg Gln Gln Asp Asp Ala Val Trp Val Phe
           115                 120                 125
   Ile Arg Gly
       130
<210> 7
   <211> 123
   <212> PRT
   <213> Leptospira biflexa serovar Patoc
<400> 7
   Met Ser Lys Phe Ile Asp Pro Asn Ile Leu Gly Lys Leu Gly Thr Leu
   1               5                   10                  15
   Ala Gln Ala Glu Ala Asp Gly Tyr Pro Phe Gly Ile Val Lys Val Asp
               20                  25                  30
   Glu Ser Gly Lys Ile Leu Leu Tyr Asn Lys Tyr Glu Ser Glu Leu Ala
           35                  40                  45
   Asn Val Pro Ile Gln Thr Ala Val Gly Lys Asn Phe Phe Thr Glu Val
       50                  55                  60
   Ala Ile Cys Thr Asn Asn Arg Ile Phe Tyr Gly Arg Phe Lys Glu Gly
   65                  70                  75                  80
   Met Ile Ser Gly Asp Leu Asp Ile Ala Phe Asn Tyr Val Phe Thr Tyr
                   85                  90                  95
   Lys Met Lys Pro Thr Asn Val Val Ile His Leu Tyr His Asp Lys Gly
               100                 105                 110
   Thr Asn Ser Asn Trp Ile Phe Val Lys Leu Arg
           115                 120
<210> 8
   <211> 130
   <212> PRT
   <213> Leptothrix cholodnii
<400> 8
   Met Asn Glu Ala Leu Pro Leu Val Phe Asp Gln Pro Asp Leu Ala Ala
   1               5                   10                  15
   Cys Ile Gly Thr Leu Ser Glu Ala Gln Leu Asp Gly Leu Gly Phe Gly
               20                  25                  30
   Val Ile Gly Phe Asp Ala Gln Gly Val Val Arg Val Tyr Asn Ala Phe
           35                  40                  45
   Glu Ser Lys Tyr Ala Gly Leu Ser Pro Gln Arg Val Leu Gly His Pro
       50                  55                  60
   Leu Phe Thr Val Val Ala Pro Cys Met Asn Asn Phe Met Val Ala Gln
   65                  70                  75                  80
   Arg Phe Glu Asp Ala Ala Ala Ser Ala Ala Ser Leu Asp Ala Thr Ile
                   85                  90                  95
   Asp Tyr Val Leu Thr Leu Arg Met Arg Pro Val Lys Val Lys Leu Arg
               100                 105                 110
   Leu Leu Ala Ala Pro Ala Thr Ala Leu Arg Tyr Val Leu Val Gln Arg
           115                 120                 125
   Pro Ala
       130
<210> 9
   <211> 123
   <212> PRT
   <213> Burkholderia phytofirmans
<400> 9
   Met Asp Asn Glu Phe Glu Ser Val Arg Ile Ala Glu Leu Ala Met Leu
   1 5 10 15
   Asp Ala Asp Arg Leu Asp Gly Val Pro Phe Gly Val Ile Gly Phe Thr
   20 25 30
   Ser Asp Ala Leu Val Thr Val Tyr Asn Ala Thr Glu Ser Lys Asn Ala
   35 40 45
   Gly Leu Arg Pro Lys Met Val Leu Gly Lys His Phe Phe Gly Glu Val
   50 55 60
   Ala Pro Cys Met Asn Asn Phe Met Val Ala Gln Arg Phe Glu Asp Glu
   65 70 75 80
   Asp Val Leu Asp Asp Ile Val Pro Tyr Val Leu Thr Leu Arg Met Arg
   85 90 95
   Pro Thr Pro Val Arg Leu Arg Leu Leu Lys Ala Thr Asp Cys Ala Thr
   100 105 110
   Arg Phe Val Leu Ile Glu Arg Arg Ala Thr Asn
   115 120
<210> 10
   <211> 156
   <212> PRT
   <213> Salinibacter ruber
<400> 10
   Met Ala Asp Ser Gln Asn Pro Tyr Ser Tyr Leu Arg Glu Asp Asp Pro
   1 5 10 15
   Asp Ser Ala Pro Gly Asp Ser Gly Asp Ala Asp Glu Pro Glu Pro Pro
   20 25 30
   Ala Thr Asp Leu Ala Phe Asp Asp Glu Gly Val Gly Glu Glu Leu Arg
           35                  40                  45
   His Val Asp Glu Asp Glu Leu Asn Ala Ala Pro Phe Gly Ile Ile Gln
       50                  55                  60
   Ile Asp Asp Ala Gly Val Val Gln Phe Tyr Asn Arg Tyr Glu Ser Asn
   65                  70                  75                  80
   Leu Ser Gly Ile Asp Pro Ala Asp Ala Val Gly Ala Asn Phe Phe Thr
                   85                  90                  95
   Glu Leu Ala Pro Cys Ser Asn Asn Pro Leu Phe Phe Gly Arg Phe Lys
               100                 105                 110
   Asp Gly Val Arg Glu Gly Gly Leu Asp Glu Tyr Phe Thr Tyr Thr Phe
           115                 120                 125
   Thr Tyr Gln Met Arg Pro Thr Leu Val Asp Val Arg Leu Tyr Arg Asp
       130                 135                 140
   Glu Ala Glu Asn Asn Trp Ile Leu Ile Gln Lys Arg
   145                 150                 155
<210> 11
   <211> 125
   <212> PRT
   <213> Idiomarina loihiensis
<400> 11
   Met Glu Ile Val Gln Phe Gly Ser Asp Asp Ile Glu Asn Thr Leu Ser
   1               5                   10                  15
   Lys Met Ser Asp Asp Lys Leu Asn Asp Ile Ala Phe Gly Ala Ile Gln
               20                  25                  30
   Leu Asp Ala Ser Gly Lys Ile Ile Gln Tyr Asn Ala Ala Glu Gly Asp
           35                  40                  45
   Ile Thr Gly Arg Asp Pro Gly Ala Val Val Gly Lys Asn Phe Phe Asn
       50                  55                  60
   Glu Val Ala Pro Cys Thr Asn Ser Pro Glu Phe Lys Gly Arg Phe Asp
   65                  70                  75                  80
   Glu Gly Val Lys Asn Gly Asn Leu Asn Thr Met Phe Glu Tyr Val Phe
                   85                  90                  95
   Asp Tyr Glu Met Gln Pro Thr Lys Val Lys Val His Met Lys Lys Ala
   100 105 110
   Leu Thr Gly Asp Thr Tyr Trp Val Phe Val Lys Arg Leu
   115 120 125
<210> 12
   <211> 130
   <212> PRT
   <213> Rhodospirillum centenum
<400> 12
   Met Pro Asp Arg Thr Thr Asp Asp Phe Gly Pro Phe Thr Glu Gln Ile
   1 5 10 15
   Arg Gly Thr Ile Asp Gly Met Gly Thr Ala Glu Phe Asp Ala Leu Pro
   20 25 30
   Val Gly Ala Ile Gln Val Asp Gly Ser Gly Val Ile His Arg Tyr Asn
   35 40 45
   Arg Thr Glu Ser Arg Leu Ser Gly Arg Ile Pro Glu Arg Val Ile Gly
   50 55 60
   Arg Asn Phe Phe Thr Glu Val Ala Pro Cys Thr Asn Ile Pro Ala Phe
   65 70 75 80
   Ser Gly Arg Phe Met Asp Gly Val Thr Ser Gly Thr Leu Asp Ala Arg
   85 90 95
   Phe Asp Phe Val Phe Asp Phe Gln Met Ala Pro Val Arg Val Gln Ile
   100 105 110
   Arg Met Gln Asn Ala Gly Val Pro Asp Arg Tyr Trp Ile Phe Val Arg
   115 120 125
   Lys Leu
   130
<210> 13
   <211> 125
   <212> PRT
   <213> Thermochromatium tepidum
<400> 13
   Met Asn Phe Glu Asp Ala Ile Asp Ile His Ala Pro Arg Arg Leu Asp
   1 5 10 15
   Ala Leu Thr Pro Asp Glu Leu Asn Arg Leu Pro Phe Gly Ala Ile Arg
   20 25 30
   Val Asp Ala Glu Gly Arg Ile Leu Phe Tyr Ser Arg Ala Leu Val Asp
   35 40 45
   Leu Ala Asn Arg Gln Val Asp Ser Val Leu Gly Arg Asn Phe Phe Ser
   50 55 60
   Glu Ile Ala Pro Cys Thr Val Val Pro Glu Phe Tyr Gly Arg Phe Arg
   65 70 75 80
   Gln Gly Val Leu Thr Gly Gln Leu His Thr Thr Phe Glu Phe Val Phe
   85 90 95
   Asp Phe Asp Met Gln Pro Val Gln Val Arg Ile Ala Met His Thr Ser
   100 105 110
   Glu Arg Pro Gly Glu Phe Trp Ile Leu Val Gln Pro Leu
   115 120 125
<210> 14
   <211> 126
   <212> PRT
   <213> Rhodopseudomonas palustris
<400> 14
   Met Asn Thr Val Asp Phe His Asp Ser Asp Leu Ala Arg Thr Ile Glu
   1 5 10 15
   Gln Leu Ala Pro Glu Gln Ile Asp Ala Leu Pro Phe Gly Val Ile Lys
   20 25 30
   Leu Asp Gly Asn Gly Ile Val Thr Val Phe Asn Arg Thr Glu Ala Ile
   35 40 45
   Glu Ser Gly Tyr Lys Ser Arg Pro Ala Leu Gly Leu Asp Phe Phe Leu
   50 55 60
   Gln Val Ala Pro Cys Met Gly Gln Pro Glu Phe Arg Gly Arg Ile Glu
   65 70 75 80
   Gln Ala Arg Gln Leu Gly Arg Val Asp Ile Glu Leu Gly Trp Val Gly
   85 90 95
   Asp Phe Ser Asp Ile Asn Arg Ser Leu Gln Val Arg Ile Gln Ser Ala
   100 105 110
   Ser Asp Gly Ser Leu Trp Ile Phe Asn Leu Arg Asp His Ala
   115 120 125
<210> 15
   <211> 146
   <212> PRT
   <213> Stigmatella aurantiaca PYP2
<400> 15
   Met Ala Pro Pro Ser Thr Ser Leu Ala Leu Ser Asn Ala Pro Pro Pro
   1 5 10 15
   Lys Thr Thr Pro Thr Asp Asp Leu Leu Arg Gln Val Glu Thr Leu Ser
   20 25 30
   Ala Ser Glu Leu Asp Ala Leu Pro Phe Gly Leu Ile Gln Leu Asp Arg
   35 40 45
   Thr Gly Arg Ile Leu Lys Phe Asn Gln Thr Glu Ala Lys Leu Ala Arg
   50 55 60
   Ile Asn Arg Glu Arg Gln Leu Gly Arg Asn Phe Phe Asp Asp Val Ala
   65 70 75 80
   Pro Cys Thr Lys Val Arg Glu Phe Tyr Gly Arg Phe Leu Asn Gly Leu
   85 90 95
   Ser Gln Arg Ser Leu Tyr Glu Thr Phe Gly Phe Ile Phe Lys Phe Asp
   100 105 110
   His Gly Trp Arg Asn Val Ala Ile Thr Met Phe Tyr Ser Glu Lys Thr
   115 120 125
   Asp Ser Val Trp Val Leu Ile Ser Gln Thr Ser Val Thr Pro Pro Pro
   130 135 140
   Ala Arg
   145
<210> 16
   <211> 130
   <212> PRT
   <213> Halorhodospira halophila PYP2
<400> 16
   Met Gly Thr Leu Ile Phe Gly Arg Gln Asp Leu Glu Asn Arg Leu Ala
   1 5 10 15
   Ala Met Thr Pro Glu Glu Ile Asp Asp Leu Pro Phe Gly Val Ile Gln
   20 25 30
   Ile Asp Gln His Gly Arg Ile Leu Leu Tyr Asn Ala Thr Glu Gly Ala
   35 40 45
   Ile Thr Gly Arg Asp Pro Glu Ala Met Ile Gly Arg Asp Phe Phe Asn
       50                  55                  60
   Asp Val Ala Pro Cys Gly His Thr Glu Ala Phe Tyr Gly Arg Phe Gln
   65                  70                  75                  80
   Glu Gly Val Arg His Gly Asp Leu Asn Glu Ile Phe Asp Tyr Thr Phe
                   85                  90                  95
   Asp Tyr Arg Met Ala Pro Thr Lys Val Arg Val His Met Lys Arg Ala
               100                 105                 110
   Leu Ser Gly Asp Thr Tyr Trp Ile Phe Val Lys Arg Ile Ser Ala Pro
           115                 120                 125
   Ala Ala
       130
<210> 17
   <211> 125
   <212> PRT
   <213> Halochromatium salexigens
<400> 17
   Met Asn Ile Val His Phe Gly Ser Asp Asp Ile Glu Asn Ser Leu Ala
   1               5                   10                  15
   Asn Met Ser Asp Gln Asp Leu Asn Gln Leu Ala Phe Gly Ala Ile Gln
               20                  25                  30
   Leu Asp Ala Ser Gly Lys Val Leu Gln Tyr Asn Ala Ala Glu Glu Gly
           35                  40                  45
   Ile Thr Gly Arg Asp Pro Lys Ser Val Ile Gly Lys Asn Phe Phe Glu
       50                  55                  60
   Asp Val Ala Pro Cys Thr Lys Ser Gln Glu Phe Gln Gly Arg Phe Lys
   65                  70                  75                  80
   Glu Gly Val Ala Asn Gly Asn Leu Ala Thr Met Phe Glu Tyr Val Phe
                   85                  90                  95
   Asp Tyr Gln Met Lys Pro Thr Lys Val Lys Val His Met Lys Lys Ala
               100                 105                 110
   Leu Val Asp Asp Ser Tyr Trp Ile Phe Val Lys Arg Leu
           115                 120                 125
<210> 18
   <211> 378
   <212> DNA
   <213> Halorhodospira halophila
<400> 18
   atggaacacg tagccttcgg tagcgaggac atcgagaaca ccctcgccaa gatggacgac      60
   ggccagctcg acggcctggc cttcggcgcc atccagctcg acggcgacgg caacatcctt     120
   cagtacaacg ccgcggaggg cgacatcacc ggccgcgacc cgaagcaggt catcggcaag     180
   aacttcttca aggacgtggc cccgtgcact gacagcccgg agttctacgg caagttcaag     240
   gaaggggtgg cctcgggcaa cctgaacacg atgttcgagt acaccttcga ttaccaaatg     300
   acgcccacga aggtgaaggt gcacatgaag aaggccctct ccggcgacag ctactgggtc     360
   ttcgtcaagc gcgtctag                                                   378
<210> 19
   <211> 375
   <212> DNA
   <213> Rhodobacter capsulatus
<400> 19
   atggaaatca ttccgttcgg gacgaacgac atcgacaaca tcctggcgcg cgagcccgcg      60
   cgtgcggaaa gcctgccgtt cggcgccgtg cttctcgacc gcatggggcg gatcgccaaa     120
   tacaacaagg ccgaggggct gatcgcgggc cgcgatccct cgacggtgat cggccgcgat     180
   ttcttcaacg agatcgcgcc ctgcgccaag ggcaagcggt tccacgggga attcctgaaa     240
   ttcaaccgca ccggccaggc caatgtgatg ctggactaca agttcaatta caagggcgcc     300
   gaagtggcgg tgaagatcca cctcaagtcc caacccgacg gccagttctg ctggctcttc     360
   gtgaagcggg cctga                                                      375
<210> 20
   <211> 375
   <212> DNA
   <213> Rhodobacter sphaeroides
<400> 20
   atggaaatca ttccctttgg cagcgccgac ctcgacaaca tccttgcccg cgagccgcag 60
   cgggccgagt atctcccctt cggcgccgtc ctgctggacc gcaccggcac gatcctgaaa 120
   tacaacaggg ccgagggtgg catcgccaac cgcaatccgg ccgacgtgat cggaaagaac 180
   ttcttcaacg agatcgcgcc ctgcgccaag ggcaagcgct ttcacggcga gttcctgcgc 240
   tttcaccaga ccggacaggt caatgtgatg ttcgattaca aattcgccta caagggcgcc 300
   aatgtcggcg tgaagatcca catgaaatcg cagcccgacg gccagagctg ctggctcttc 360
   gtcaaacggg tctga 375
<210> 21
   <211> 378
   <212> DNA
   <213> Rhodothalassium salexigens
<400> 21
   atggaaatga tcaaattcgg ccaggacgac atcgagaacg ccatggcgga tatgggcgac 60
   gcgcagatcg acgacctggc ttttggcgcc attcaactgg acgagaccgg cacgatcctg 120
   gcctataacg cggccgaggg cgaactaacc ggccgcagtc cccaagacgt gatcggcaag 180
   aacttcttca aggacatagc gccgtgcacc gacaccgagg aattcggcgg ccggttccgc 240
   gaaggggtgg ccaatggcga cctgaacgcg atgttcgaat atgtcttcga ctatcagatg 300
   cagccgacca aggtgaaggt gcacatgaag cgcgctatca ccggcgacag ctactggatc 360
   ttcgtcaagc gtgtctga 378
<210> 22
   <211> 408
   <212> DNA
   <213> Stigmatella aurantiaca
<400> 22
   atgcggcatg ggattctgga agcggagtcg ctgacagagg atcggctggg ccagctgtcg 60
   cccgaggagt ttgatgcgtt gccgtttggc gccatcaagc tggacgcgga gggacgggtg 120
   ctcatctaca acgcggcgga gtccgcgttc tcccggcgca agcccgtgtc cgtgctcggc 180
   cgccgcttct tcgaggaggt tgccccttgt accaacgtgg ccagcttccg ggggcgtttc 240
   gacacgctcg tcgagcgagg gcatggcacg gagagcttcg acttccagtt ccgattccgc 300
   tggggcacac gcaacgtgcg catccggctg atggtgctgg gggacggttc ccgctgggtc 360
   ttcgtgacgg cggtgctgac ggcgctgatc cccctcggcg agggctga 408
<210> 23
   <211> 396
   <212> DNA
   <213> Sorangium cellulosum
<400> 23
   atgggttcgg aggagcggtc cacagccggt gagttcgagt tcgatatcgg ggtgttcaac 60
   ctggatgaac gcggcctgga cgcgcagccc ttcgggatca tccggctgga tcgcgagggc 120
   acggtgctct cctacaacct ctacgaggaa cgtcaggcgc gccggaatcg gcaggacgtg 180
   atagggaaga acttcttcac cgacatcgcg ccctgcagcc gggtgaaggc gttccacggc 240
   cgcttcctgg ccggggtcga gcagcgcgag ctcaaggcca ccttcggctt cgtgttccac 300
   ttccctcaca agacacggca cgtcgacgtc tcgctgttct acaaagcggc cgcgcgacag 360
   caggacgatg cggtctgggt gttcatccga ggttga 396
<210> 24
   <211> 372
   <212> DNA
   <213> Leptospira biflexa serovar Patoc
<400> 24
   atgagcaaat ttatagaccc aaatatttta ggaaaactcg gtaccttggc acaagcggaa 60
   gctgacggat acccgtttgg aattgtcaaa gtagacgagt cgggtaaaat tttattgtat 120
   aacaaatacg aatccgaact tgccaatgtt cccatccaaa cagccgtagg gaaaaacttt 180
   tttacggaag tggctatttg tacgaacaac cgaatcttct atggaaggtt taaagaaggg 240
   atgatttcgg gagatttgga catcgcattc aattatgtat tcacttacaa aatgaaacca 300
   accaatgttg tgatccatct ctaccatgac aaagggacca attccaattg gatttttgtc 360
   aaactcagat aa 372
<210> 25
   <211> 393
   <212> DNA
   <213> Leptothrix cholodnii
<400> 25
   atgaatgaag cgctgccgct ggtgttcgac cagcctgatc tggctgcctg catcggcacg 60
   ctgagcgagg cgcagctcga tggcctcggc ttcggtgtga tcggcttcga tgcgcagggt 120
   gtggtgcgtg tctacaacgc cttcgagtcg aaatatgccg ggctgtcgcc gcagcgggtg 180
   ttgggccatc cgctgttcac cgtggtggca ccgtgcatga acaacttcat ggtcgcccag 240
   cgcttcgaag acgccgccgc cagcgccgcc agcctcgacg ccaccatcga ctacgtgctg 300
   acgctgcgca tgcgcccggt caaggtcaag ctccgattgc tggccgcgcc tgccacggcc 360
   ttgcgctacg tgctcgtgca acgcccggcc tga 393
<210> 26
   <211> 372
   <212> DNA
   <213> Burkholderia phytofirmans
<400> 26
   gtggacaacg aattcgaaag tgtgcggata gcagaactgg ccatgctgga cgcggaccgg 60
   ttagacgggg tgccatttgg cgtcatcgga tttacctcgg atgcacttgt cacggtctat 120
   aacgctactg aatcgaagaa cgccggattg cggccaaaga tggtgttagg gaagcatttc 180
   ttcggagagg tcgcaccgtg catgaacaat ttcatggtgg cgcagcgctt cgaagatgag 240
   gacgtacttg acgacatcgt tccctacgtt ttgaccttgc gcatgcgtcc gacgccggtg 300
   cggctcaggc tcctgaaagc cacggattgc gcgacacggt ttgtcctgat tgagcgaagg 360
   gcgacaaatt ga 372
<210> 27
   <211> 471
   <212> DNA
   <213> Salinibacter ruber
<400> 27
   atggctgact ctcagaatcc gtactcgtac ctgcgtgaag acgacccgga ctccgccccc 60
   ggcgactcgg gcgacgccga cgagcctgag ccgccggcaa cggacctcgc gttcgacgac 120
   gagggggtgg gggaggagct gcgtcacgtc gacgaggacg agctaaatgc ggccccgttc 180
   ggcatcattc agattgacga cgcgggggtc gtgcagttct acaaccgcta cgagtcgaac 240
   ctaagcggca tcgatccggc agacgccgtc ggggccaact tcttcacgga gctggcgccg 300
   tgcagcaaca acccgctgtt cttcggccgc ttcaaggacg gggttcggga gggggggctc 360
   gacgagtact tcacgtacac gttcacctac cagatgcgcc ccaccctcgt cgacgtgcgg 420
   ctctaccgcg acgaggcgga gaacaactgg atcctgattc agaagcggtg a 471
<210> 28
   <211> 378
   <212> DNA
   <213> Idiomarina loihiensis
<400> 28
   atggagattg ttcaattcgg ttctgatgat atcgaaaaca ctttatctaa aatgtcagat 60
   gacaagttaa acgacattgc ttttggcgct attcagttag acgctagcgg taaaattatt 120
   caatacaacg ccgccgaagg cgacattacc ggtcgtgatc cgggcgctgt cgttggcaaa 180
   aactttttta acgaagtggc accatgcacc aacagccctg agtttaaagg acgcttcgac 240
   gaaggcgtta aaaacggcaa tttaaacacc atgtttgaat atgtttttga ctacgaaatg 300
   caaccgacca aagttaaagt tcatatgaaa aaagctttaa ccggcgacac ctactgggta 360
   ttcgttaagc gactataa 378
<210> 29
   <211> 390
   <212> DNA
   <213> Rhodospirillum centenum
<400> 29
   gtgccggacc ggaccaccga cgatttcggc cccttcaccg agcagatccg cggcaccatc 60
   gacgggatgg ggacggccga gttcgatgcc ttgccggtcg gcgccatcca ggtggacggc 120
   agcggggtca tccaccgtta caacaggacg gaaagccggc tcagcggccg catccccgaa 180
   cgggtcatcg ggcgcaactt cttcaccgag gtggcgccct gcacgaacat cccggccttc 240
   agcggccgct tcatggacgg tgtgacctcc ggcacgctgg atgcccgctt cgacttcgtc 300
   ttcgatttcc agatggcgcc ggtgcgggtg cagatccgca tgcagaacgc gggcgtgccc 360
   gaccgctact ggatcttcgt gcgcaagctg 390
<210> 30
   <211> 31
   <212> DNA
   <213> Artificial Sequence
<220>
   <223> Forward Primer
<400> 30
   cggagatata catatgagag gatcgcatca c 31
<210> 31
   <211> 21
   <212> DNA
   <213> Artificial Sequence
<220>
   <223> Reverse Primer
<400> 31
   gctaattaag cttctagacg c 21

## Description

### Technical Field

The present invention relates to a method for fluorescently labeling a protein.

### Background Art

Recently, bioimaging for observing the functions of biomolecules such as proteins inside living organisms has been receiving attention. The observation of the biomolecule functions is being conducted widely by labeling biomolecules with fluorescence to visualize the localization or movement of the biomolecules inside living organisms. A general method for labeling biomolecules with fluorescence is one using a genetic engineering procedure to allow a fluorescent protein to express as a fusion protein of a target biomolecule (protein). This fluorescent protein has disadvantages in, for example, its large molecular size and a difficulty in observing near-infrared fluorescence with a high tissue penetration. Therefore, attention is focused on small organic molecules having smaller molecular sizes than that of a fluorescent protein and excellent fluorescent properties. With respect to technologies for labeling target proteins with small organic molecules, there are commercialized labeling kits in which HaloTag (registered trademark) (Promega KK) (see, for example, Non-Patent Document 1) and SNAP-tag (registered trademark) (see, for example, Non-Patent Document 2) are used. These labeling methods each use an enzyme reaction to label target biomolecules with fluorescence. Therefore, the specificity in labeling target biomolecules with small organic molecules is very high but fluorescence derived from the small organic molecules that are not bound to the biomolecules is observed as background signals. In order to reduce the background signals, after being fluorescently labeled with the small organic molecules, washing is necessary to remove unreacted small organic molecules. However, it is generally difficult to wash biomolecules present in a cell or in vivo and it is often not possible to wash them at all. Accordingly, in order to observe the functions ofbiomolecules in a cell or in vivo, the above-mentioned labeling methods cannot be considered versatile.

### Prior Art Documents

### Patent Documents

Patent Document 1: US2004/0029230 discloses DNA strands useful for the production of parahydroxycinnamic acid and photoactive yellow protein in a suitable host expression system.

### Non-Patent Documents

Non-Patent Document 1: Qureshi, M. H. et al., J. Biol. Chem., 2001, 276, p.46422-8
Non-Patent Document 2: Proc. Natl. Acad. Sci. USA 2004, 101, p.9955-9959.
Non-Patent Document 3: Takakusa et al., Anal. Chem. 2001, 73, p. 939-942 discloses an intramolecular fluorescence energy transfer system with coumarin donor included in β -cyclodextrin.
Non-Patent Document 4: van der Horst et al., Photochem. Photobio. Sci., 2007, 6:571-579, discloses a study on the binding, tuning and mechanical function of the 4-hydroxy-cinnamic acid chromophore in photoactive yellow protein.

### Disclosure of Invention

### Problem to be Solved by the Invention

Therefore, the present invention is intended to provide a method for fluorescently labeling a protein, which can be used for labeling a biomolecule (a protein) with fluorescence in a cell or in vivo.

### Means for Solving Problem

The present invention is a method for fluorescently labeling a protein that includes obtaining a fusion protein of a target protein to be labeled and a photoactive yellow protein (PYP) or a PYP-derived protein, and fluorescently labeling the target protein by reacting the fusion protein with a compound represented by Formula (I) below or a salt thereof,
wherein in the compound represented by Formula (I) or the salt thereof, a coumarin skeleton in Formula (A) is intramolecularly associated with a group X and as a result, fluorescence derived from the group X is suppressed, and
when in the compound represented by Formula (I) or a salt thereof, the coumarin skeleton in Formula (A) is bound to the PYP or the PYP-derived protein contained in the fusion protein, the coumarin skeleton in Formula (A) is intramolecularly dissociated from the group X, and thereby fluorescence derived from the group X can be emitted.

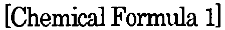

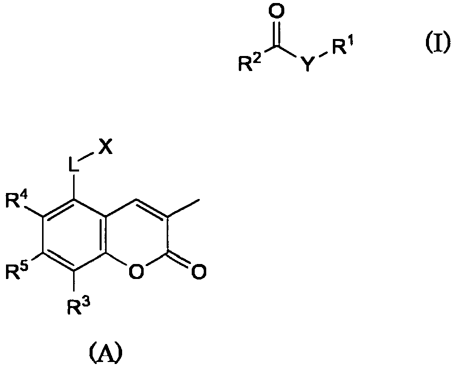

In Formula (I),
Y is an oxygen atom or a sulfur atom,
R¹ is a lower alkyl group, a lower alkyl group substituted with halogen, an aryl group, an aryl group substituted with at least one selected from the group consisting of hydroxy, halogen, and lower alkoxy, a lower aralkyl group, or a lower aralkyl group substituted with at least one selected from the group consisting of hydroxy, halogen, and lower alkoxy, and
R² is a group represented by Formula (A) including the coumarin skeleton,
wherein in Formula (A),
X is a fluorescent group,
L is a linker for X,
R³ and R⁴ are each independently a hydrogen atom, a halogen atom, a lower alkyl group, a hydroxy group, a lower alkoxy group, or a lower alkyl group substituted with halogen,
R⁵ is a hydroxy group, an amino group, an amino group that has been mono- or di-substituted with lower alkyl, or a lower alkoxy group, and
wherein the amino acid sequence of the PYP or PYP-derived protein is one selected from the group consisting of:
1) amino acid sequences represented by SEQ ID NOS: 1 to 17 of the sequence listing,
2) amino acid sequences represented by SEQ ID NOS: 1 to 17 of the sequence listing in which the 1st to 24th, 25th, 26th, or 27th amino acids have been deleted,
3) amino acid sequences in which one to several amino acids of the amino acid sequences of either 1) or 2) described above have been deleted, substituted, or added, wherein when the fusion protein binds to the coumarin skeleton in Formula (A), the coumarin skeleton in Formula (A) is intramolecularly dissociated from the group X in the compound represented by Formula (I), and thereby fluorescence derived from the group X can be emitted, and
4) amino acid sequences having a homology of at least 70% with the amino acid sequences of either 1) or 2) described above, wherein when the fusion protein binds to the coumarin skeleton in Formula (A), the coumarin skeleton in Formula (A) is intramolecularly dissociated from the group X in the compound represented by Formula (I), and thereby fluorescence derived from the group X can be emitted.

### Effects of the Invention

In the method for fluorescently labeling a protein according to the present invention, high fluorescence is exhibited only when a target protein has been labeled. Therefore, the fluorescence background can be minimized without removing small organic molecules that are not labeling proteins. Accordingly, highly accurate fluorescence imaging can be carried out. Furthermore, since it is not necessary to remove unreacted small organic molecules, the method is suitable for observing biomolecules in vivo.

### Brief Description of Drawings

[FIG. 1] FIG. 1(a) shows a CBB-stained image obtained after the reaction between a PYP and a compound FCTP and FIG. 1(b) shows a fluorescent image obtained after the reaction between the PYP and the compound FCTP
[FIG. 2] FIG. 2(a) shows a CBB-stained image obtained after the reaction between a PYP and a compound FCTP in a cell lysate and FIG. 2(b) shows a fluorescent image obtained after the reaction between the PYP and the compound FCTP in the cell lysate.
[FIG. 3(a)] FIG. 3(a) shows time-dependent fluorescence spectra of 8 pM of compound FCTP in the presence of 5 µM of PYP.
[FIG. 3(b)] FIG. 3(b) shows time-dependent excitation spectra of 8 µM of compound FCTP in the presence of 5 µM of PYP.
[FIG. 3(c)] FIG. 3(c) shows fluorescence spectra (excitation at 500 nm) of 8 µM of compound FCTP.
[FIG. 4] FIG. 4 shows fluorescence microscopic images illustrating a labeling reaction using a FCTP in a cultured cell.
[FIG. 5] FIG. 5 shows the procedure of a labeling reaction using a CATP in a cultured cell.
[FIG. 6] FIG. 6 shows fluorescence microscopic images illustrating a labeling reaction using a CATP in a cultured cell.

### Description of the Invention

A photoactive yellow protein (PYP) is a photoreceptor protein isolated from purple photosynthetic bacteria, Ectothiorhodospira halophila (Halorhodospira halophila). Since the purple photosynthetic bacteria are eubacteria, a PYP-derived endogenous protein is unlikely to adversely affect fluorescence labeling when a PYP is used in a cell. Furthermore, the PYP consists of 125 amino acid residues (SEQ ID NO: 1 of the sequence listing) and is a relatively small protein (14 kDa). In the PYP, a chromophore, p-coumaric acid, binds to the 69th cysteine residue through a thioester bond. It is known that various analogs other than p-coumaric acid each are bound to a PYP (see, for instance, compounds shown in Scheme 1 below as well as, for example, Cordgunke et al., Proc. Natl. Acad. Sci, USA, 1998, 95, pp7396-7401 and Koon et al., The Journal of Biological Chemistry, 1996, 271, pp. 31949-31956).

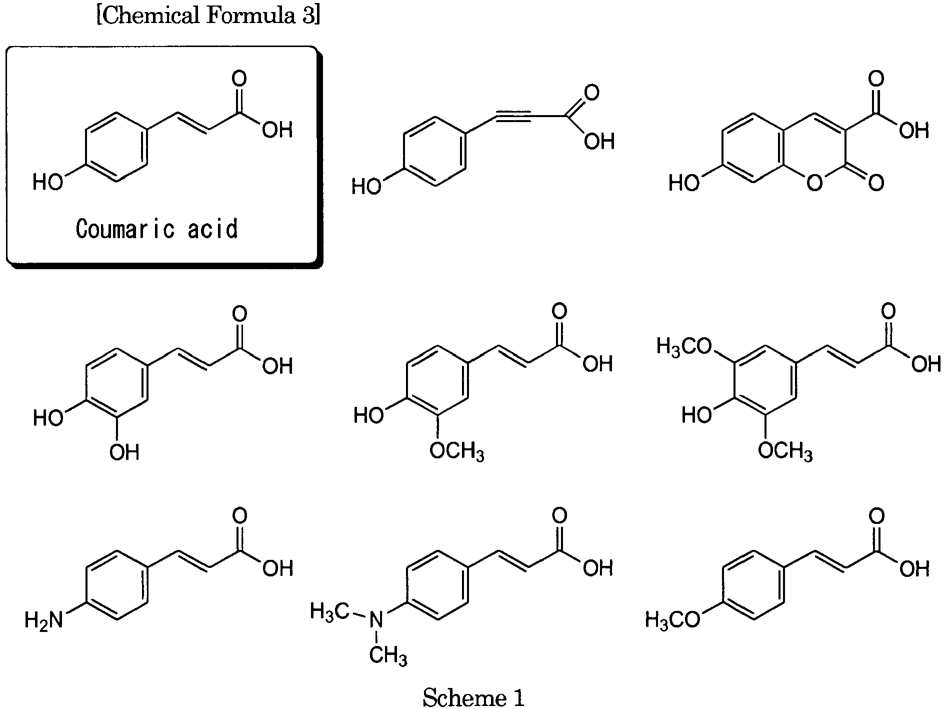

According to the labeling method of the present invention, in a compound represented by Formula (I) or a salt thereof, the coumarin skeleton in Formula (A) and the group X, which are bound to each other through the linker L, are intramolecularly associated and as a result, the fluorescence derived from the group X is suppressed (see Scheme 2). In the labeling method of the present invention, a fusion protein obtained by fusing a PYP or PYP-derived protein as defined in the claims with a target protein to be labeled is reacted with the compound of Formula (I) or the salt thereof. With respect to the PYP or PYP-derived protein of the fusion protein as defined in the claims, when the coumarin skeleton in Formula (A) of the compound of Formula (I) is bound to the PYP or PYP-derived protein contained in the fusion protein as defined in the claims, a binding site of, for example, the PYP has a space into which only the coumarin skeleton in formula (A) can get and the group X cannot get into the binding site of, for example, the PYP. As a result, the coumarin skeleton in Formula (A) is intramolecularly dissociated from the group X and therefore fluorescence derived from the group X can be emitted (see Scheme 2). In other words, the compound of Formula (I) or the salt thereof that is not bonded to the fusion protein has very low fluorescence intensity. That is, it does not emit fluorescence, which allows the background signals to be low in the labeling method of the present invention. As a result, after the target protein to be labeled (biomolecule) is labeled with the compound of Formula (I), even without washing, the target protein to be labeled can be detected accurately without any disturbance from background signals. Furthermore, the PYP or PYP-derived protein as defined in the claims that is used in the labeling method of the present invention has a relatively low molecular weight, approximately 14 kDa, and therefore is unlikely to affect the target protein to be labeled, which is an advantage. Moreover, since the PYP or PYP-derived protein as defined in the claims or the coumarin derivative or the coumaric acid derivative is a substance that does not exist in an animal cell, it is unlikely to affect the label, which is an advantage.

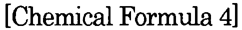

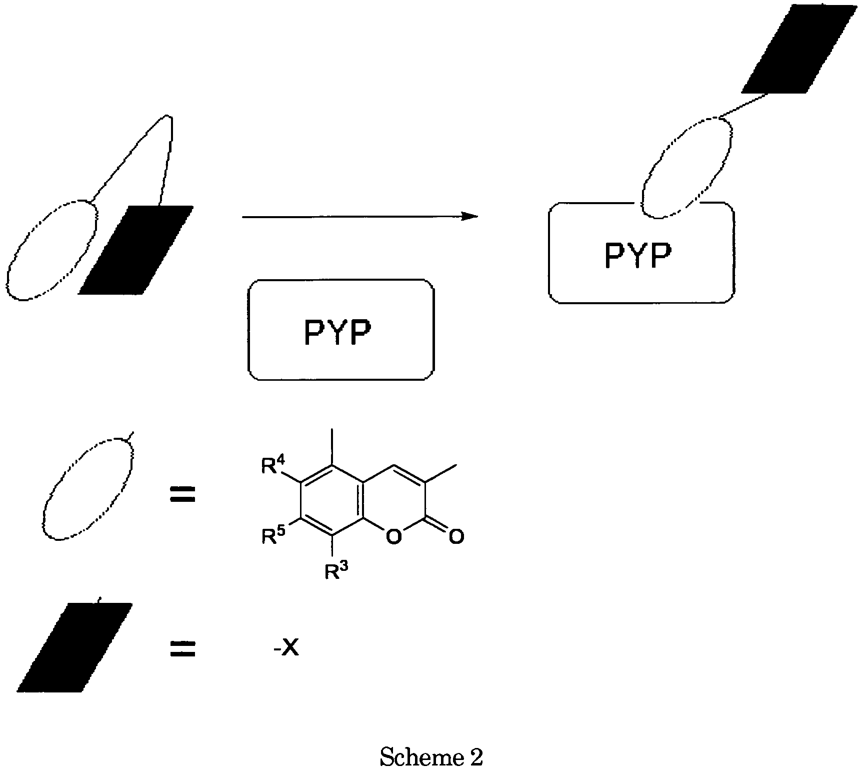

Examples of the amino acid sequence of the PYP include amino acid sequences represented by SEQ ID NOS: 1 to 17 of the sequence listing. Examples of the polynucleotide for coding the PYP include base sequences represented by SEQ ID NOS: 18 to 29 of the sequence listing.

In order to obtain a plasmid or vector that can express a fusion protein, for example, a polynucleotide that codes a target protein to be labeled or a polynucleotide that codes a PYP or PYP-derived protein as defined in the claims can be used to prepare such a plasmid or vector according to an ordinary method. In the present invention, the vector is a polynucleotide, preferably a polynucleotide for transfecting a cell with a polynucleotide for coding the fusion protein, and embraces a plasmid. The vector may be a plasmid vector or a viral vector. A person skilled in the art suitably can select the sequence that allows the fusion protein to be expressed, according to the type of the cell to be transfected. The plasmid and the vector may contain a sequence (for example, an expression-inducing promoter or a regulatory sequence) that adjusts expression of the fusion protein.

The fusion protein can be expressed in a cell by using a polynucleotide for coding the fusion protein or a plasmid or vector that can express the fusion protein, according to an ordinary method.

An expressed fusion protein can be isolated according to an ordinary method.

As described above, in the compound of Formula (I) that is used in the fluorescently labeling method of the present invention, Y is an oxygen atom or a sulfur atom,
R¹ is a lower alkyl group, a lower alkyl group substituted with halogen, an aryl group, an aryl group substituted with at least one selected from the group consisting of hydroxy, halogen, and lower alkoxy, a lower aralkyl group, or a lower aralkyl group substituted with at least one selected from the group consisting of hydroxy, halogen, and lower alkoxy, and
R² is a group represented by Formula (A) including the coumarin skeleton,
wherein in Formula (A),
X is a fluorescent group,
L is a linker for X,
R³ and R⁴ are each independently a hydrogen atom, a halogen atom, a lower alkyl group, a hydroxy group, a lower alkoxy group, or a lower alkyl group substituted with halogen, and
R⁵ is a hydroxy group, an amino group, an amino group that has been mono- or di-substituted with lower alkyl, or a lower alkoxy group.

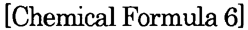

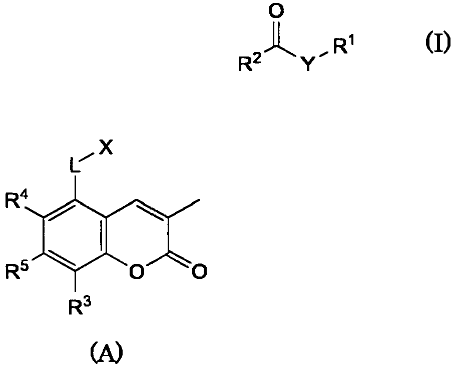

In the present invention, the fluorescent group denotes a group having properties in which when it is irradiated with, for example, visible light, ultraviolet rays, or X-rays, it absorbs energy thereof, thereby electrons are excited, and it releases extra energy as an electromagnetic wave when it returns to the ground state. Specifically, examples of the fluorescent group include groups derived from, for example, fluorescein, a xanthene dye, a cyanine dye, acridine, isoindole, a dansyl dye, aminophthalic hydrazide, aminophthalimide, aminonaphthalimide, aminobenzofuran, aminoquinoline, or dicyanohydroquinone, or a luminous rare earth complex. Preferably, the fluorescent group is a group derived from fluorescein or a xanthene dye.

In the present invention, the linker L for X is a group for binding the group X and the coumarin skeleton of the group of Formula (A). Preferably, the linker L is flexible and long to some extent so that the coumarin skeleton of Formula (A) is intramolecularly associated with the group X. In order to be flexible, the linker L is preferably one having a chain structure. Furthermore, the length of the linker L is, for example, 8Ǻ to 25Ǻ, preferably 10Ǻ to 20Ǻ, and more preferably 12Ǻ to 18Ǻ. Specifically, examples of L include -(CH₂)ₙ₁O(CH₂)ₙ₂-, -(CH₂)ₙ₁CONR¹¹(CH₂)n₂-, -(CH₂)ₙ₁NR¹¹CO(CH2)n₂-, -(CH₂)ₙ₁NR¹¹CONR¹²(CH₂)ₙ₂-, -(CH₂)ₙ₁NR¹¹CSNR¹²(CH₂)ₙ₂-, -(CH₂)ₙ₁CONR¹¹(CH₂)ₙ₃CONR¹²(CH₂)ₙ₂-, -(CH₂)ₙ₁NR¹¹CO(CH₂)n₃NR¹²CO(CH₂)n₂- , -(CH₂)ₙ₁-, -(CH₂)ₙ₁S(CH₂)ₙ₂-, -(CH₂)ₙ₁NR¹¹(CH₂)ₙ₂-, -(CH₂)ₙ₁CO₂(CH)ₙ₂-, and -(CH₂)ₙ₁O(CH₂)ₙ₂O(CH₂)ₙ₃O(CH₂)ₙ₄- as well as 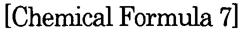 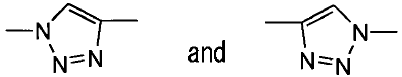 and those obtained by combining two or more of them. In the above-mentioned formulae, n1, n2, and n3 are each independently an integer of 0 or 1 to 5, and R¹¹ and R¹² each are a hydrogen atom or a lower alkyl group. The structure of the linker for X is not limited as long as the linker is not cleaved under conditions in the method for fluorescently labeling a protein of the present invention. The aforementioned linker L is preferably a group represented by Formula (a), Formula (b), Formula (c), or Formula (d) below.

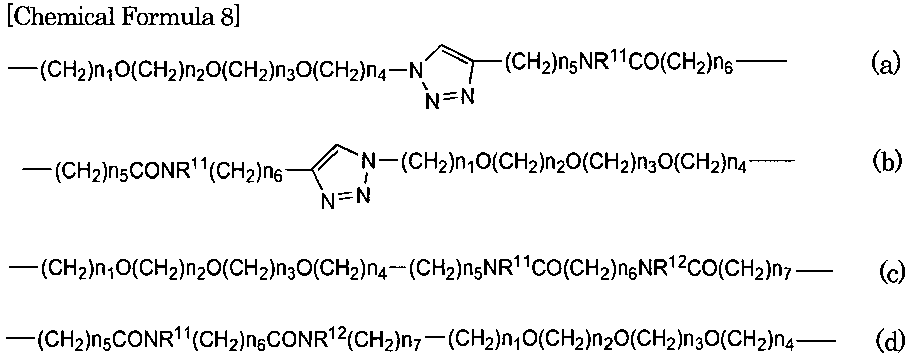

In Formula (a) and Formula (b), n1, n2, n3, n4, n5, and n6 are each independently an integer of 0 or 1 to 5, and R¹¹ is a hydrogen atom or a lower alkyl group, and
in Formula (c) and Formula (d), n1, n2, n3, n4, n5, n6, and n7 are each independently an integer of 0 or 1 to 5, and R¹¹ and R¹² are each independently a hydrogen atom or a lower alkyl group.

In the compound of Formula (I), R¹, R², and a group of Formula (A) each may have at least one chiral center. Therefore, the compound of Formula (I) may be present as a racemate or a pure stereoisomer. In each case, the present invention includes both mixtures thereof and respective isomers thereof.

In the present invention, the term "lower" denotes 1 to 6 carbon atoms and preferably 1 to 4 carbon atoms unless otherwise indicated.

In the present invention, examples of the "halogen atom" or "halogen" include a fluorine atom, a chlorine atom, a bromine atom, and an iodine atom.

In the present invention, examples of the "lower alkyl group" or "lower alkyl" include those having 1 to 6 carbon atoms. Specific examples of the lower alkyl group include linear or branched alkyl groups such as methyl, ethyl, n-propyl, i-propyl, n-butyl, i-butyl, sec-butyl, t-butyl, n-pentyl, i-pentyl, sec-pentyl, t-pentyl, 2-methylbutyl, n-hexyl, 1-methylpentyl, 2-methylpentyl, 3-methylpentyl, 4-methylpentyl, 1-ethylbutyl, 2-ethylbutyl, 1,1-dimethylbutyl, 2,2-dimethylbutyl, 3,3-dimethylbutyl, and 1-ethyl-1-methylpropyl, and preferably those having 1 to 3 carbon atoms.

In the present invention, examples of the "lower alkoxy group" or "lower alkoxy" include alkyloxy groups having 1 to 6 carbon atoms. Specific examples of the lower alkoxy groups include linear or branched alkyloxy groups such as methoxy, ethoxy, n-propyloxy, i-propyloxy, n-butyloxy, i-butyloxy, sec-butyloxy, t-butyloxy, n-pentyloxy, i-pentyloxy, sec-pentyloxy, t-pentyloxy, 2-methylbutoxy, n-hexyloxy, i-hexyloxy, t-hexyloxy, sec-hexyloxy, 2-methylpentyloxy, 3-methylpentyloxy, 1-ethylbutyloxy, 2-ethylbutyloxy, 1,1-dimethylbutyloxy, 2,2-dimethylbutyloxy, 3,3-dimethylbutyloxy, and 1-ethyl-1-methylpropyloxy, and preferably alkyloxy groups having 1 to 3 carbon atoms.

In the present invention, examples of the "aryl group" include those having 6 to 10 carbon atoms. Specific examples of the aryl group include a phenyl group and a naphthyl group and preferably a phenyl group.

In the present invention, examples of the "lower aralkyl group" include aryl-substituted lower alkyl groups. Specific examples of the lower aralkyl group include benzyl(phenylmethyl), phenethyl(phenylethyl), 3-phenylpropyl, 1-naphthylmethyl, and 2-(1-naphthyl)ethyl, and preferably a benzyl group.

In the present invention, examples of the "lower alkyl group substituted with halogen" include fluoromethyl, difluoromethyl, trifluoromethyl, chloromethyl, dichloromethyl, trichloromethyl, bromomethyl, dibromomethyl, tribromomethyl, 1-fluoroethyl, 1-chloroethyl, 1-bromoethyl, 2-fluoroethyl, 2-chloroethyl, 2-bromoethyl, 1,2-difluoroethyl, and tolufluoromethyl.

In the present invention, examples of the "amino group that has been mono- or di-substituted with lower alkyl" include methylamino, ethylamino, n-propylamino, i-propylamino, n-butylamino, dimethylamino, and diethylamino.

In the present invention, examples of the "aryl group substituted with at least one selected from the group consisting of hydroxy, halogen, and lower alkoxy' include 4-hydroxyphenyl, 4-chlorophenyl, 4-bromophenyl, 4-fluorophenyl, 2,6-dichlorophenyl, 4-methoxyphenyl, 3,4-dimethoxyphenyl, and 2-hydroxynaphthyl.

In the present invention, examples of the "lower aralkyl group substituted with at least one selected from the group consisting of hydroxy, halogen, and lower alkoxy" include 4-hydroxybenzyl, 2-chlorobenzyl, 2-bromobenzyl, and 4-methoxybenzyl.

The salt of the compound of Formula (I) may be a salt with acid or base. Examples of such a salt include salts of alkali metal such as sodium and potassium, salts of alkaline earth metal such as calcium and magnesium, salts with inorganic bases such as ammonium salts, salts with organic amine such as triethylamine, pyridine, picoline, ethanolamine, triethanolamine, dicyclohexylamine, and N,N'-dibenzylethyleneamine, salts with inorganic acid such as hydrochloric acid, hydrobromic acid, sulfuric acid, and phosphoric acid, salts with organic carboxylic acid such as formic acid, acetic acid, trifluoroacetic acid, maleic acid, and tartaric acid, addition salts with sulfonic acid such as methanesulfonic acid, benzenesulfonic acid, and p-toluenesulfonic acid, salts with bases such as basic or acidic amino acid, such as arginine, aspartic acid, and glutamic acid, as well as acid addition salts.

The compound of Formula (I) may be in the form of a solvate and this also is included in the range of the present invention. Examples of the solvate include preferably a hydrate and an ethanolate.

The compound of Formula (I) that is used in the fluorescently labeling method of the present invention may be produced in house by reference to known documents of conventional techniques or may be obtained commercially

Obtaining a fusion protein of a target protein to be labeled and a PYP or PYP-derived protein as defined in the claims, which is used in the fluorescently labeling method of the present invention, may include, for example, obtaining a polynucleotide for coding the fusion protein, obtaining a plasmid or vector that can express the fusion protein, expressing the fusion protein in a cell, or isolating the fusion protein thus expressed. With, for example, a polynucleotide for coding a target protein to be labeled or a polynucleotide for coding a PYP or PYP-derived protein as defined in the claims being used for the polynucleotide for coding the fusion protein, a plasmid or vector that can express a fusion protein can be prepared according to the ordinary method.

In the method for fluorescently labeling a protein of the present invention, a step of reacting the fusion protein with the compound of Formula (I) may be carried out in a cell or in vivo where the fusion protein is expressed or may be carried out in vitro using an isolated fusion protein. When carried out in vitro, labeling may be carried out, for example, in a buffer (pH 6.8 to 8.5) at a temperature of 20 to 37°C.

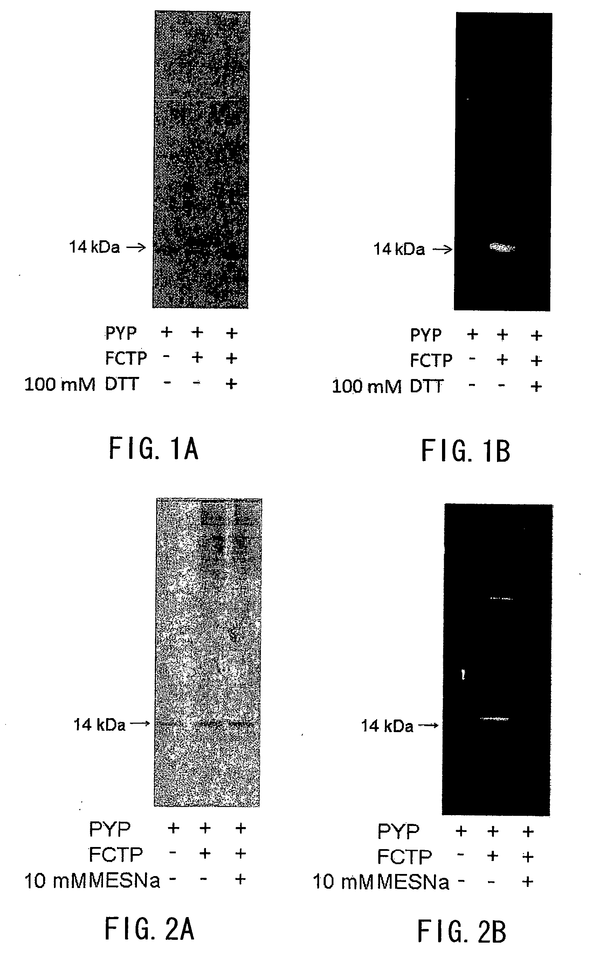

The amino acid sequences represented by SEQ ID NOS: 1 to 17 of the sequence listing are those of PYPs that are present in 15 types of purple bacteria. Furthermore, according to the document, Kumauchi et al. Photochemistry and Photobiology, 2008, 84:956-969, the PYPs of the purple bacteria are known to exhibit ligand-binding affinity even when the 24th to 27th amino acid sequences at the N terminus are deleted. A person skilled in the art can easily determine the sites that can be deleted at the N terminus with reference to the multiple alignment shown in FIG. 2(a) in the document described above.

The fusion protein of the target protein to be labeled and the PYP or PYP-derived protein as defined in the claims can be obtained by a method including at least one or all of the steps of: obtaining a polynucleotide for coding the fusion protein, obtaining a plasmid or vector that can express the fusion protein, expressing the fusion protein in a cell, or isolating the fusion protein thus expressed. That is, obtaining the fusion protein includes not only isolating the protein but also expressing the fusion protein in a cell or in vivo.

The present invention also is a composition to be used for a method of the present invention containing a compound represented by Formula (I) or a salt thereof.

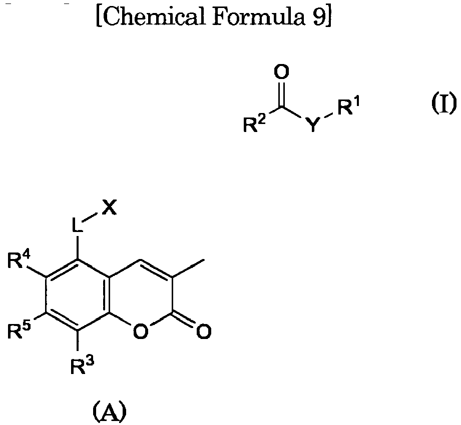

In Formula (I),
Y is an oxygen atom or a sulfur atom,
R¹ is a lower alkyl group, a lower alkyl group substituted with halogen, an aryl group, an aryl group substituted with at least one selected from the group consisting of hydroxy, halogen, and lower alkoxy, a lower aralkyl group, or a lower aralkyl group substituted with at least one selected from the group consisting of hydroxy, halogen, and lower alkoxy, and
R² is a group represented by Formula (A) including a coumarin skeleton
wherein in Formula (A),
X is a fluorescent group,
L is a linker for X,
R³ and R⁴ are each independently a hydrogen atom, a halogen atom, a lower alkyl group, or a lower alkyl group substituted with halogen, and
R⁵ is a hydroxy group, an amino group, an amino group that has been mono- or di-substituted with lower alkyl, or a lower alkoxy group.

Furthermore, the present invention is a kit for a method for fluorescently labeling a protein. The kit includes a plasmid or vector to be used for a method of the present invention and a composition to be used for a method of the present invention containing a compound represented by Formula (I) or a salt thereof, wherein
the plasmid or vector is one for cloning a polynucleotide for coding a fusion protein of a target protein to be labeled and a PYP or PYP-derived protein as defined in the claims or one for expressing the fusion protein, and
the plasmid or vector includes a base sequence selected from the group consisting of:
1) base sequences represented by SEQ ID NOS: 18 to 29 of the sequence listing,
2) base sequences represented by SEQ ID NOS: 18 to 29 of the sequence listing in which base sequences have been deleted corresponding to deletion of the 1st to 24th, 25th, 26th, or 27th at the N terminus of the amino acid sequences that are coded with the aforementioned base sequences,
3) base sequences in which one to several bases of the base sequences of either 1) or 2) described above have been deleted, substituted, or added, wherein the base sequences code amino acid sequences in which when the fusion protein binds to the coumarin skeleton in Formula (A), the coumarin skeleton in Formula (A) is intramolecularly dissociated from the group X in the compound represented by Formula (I) of the present invention, and thereby fluorescence derived from the group X can be emitted, and
4) base sequences that have a homology of at least 70% with the base sequences of either 1) or 2) described above, wherein the base sequences code amino acid sequences in which when the fusion protein binds to the coumarin skeleton in Formula (A), the coumarin skeleton in Formula (A) is intramolecularly dissociated from the group X in the compound represented by Formula (I) of the present invention, and thereby fluorescence derived from the group X can be emitted. The base sequences represented by SEQ ID NOS: 18 to 29 of the sequence listing are those for coding amino acid sequences of PYPs represented by SEQ ID NOS: 1 to 12 of the sequence listing, respectively. Deletion of 5'-side base sequences in the above-mentioned base sequences 2) is the same as described above.

Preferably, the kit for a method for fluorescently labeling a protein of the present invention further includes a host cell to be transfected with the plasmid or vector and an instruction manual for the kit.

Preferably, in the method, composition, and kit of the present invention,
Y is a sulfur atom,
R¹ is an aryl group or an aryl group substituted with at least one selected from the group consisting of hydroxy, halogen, and lower alkoxy, and
R² is a group represented by Formula (A),
wherein in Formula (A),
X is a group represented by Formula (i),
L is a group represented by Formula (a), Formula (b), Formula (c), or Formula (d),
R³ and R⁴ each are a hydrogen atom, and
R⁵ is hydroxy
wherein in Formula (a) and Formula (b),
n1, n2, n3, n4, n5, and n6 are each independently an integer of 0 or 1 to 5, and
R¹¹ is a hydrogen atom or a lower alkyl group, and
in Formula (c) and Formula (d),
n1, n2, n3, n4, n5, n6, and n7 are each independently an integer of 0 or 1 to 5, and R¹¹ and R¹² are each independently a compound of Formula (I), which is a hydrogen atom or a lower alkyl group, or a salt thereof.

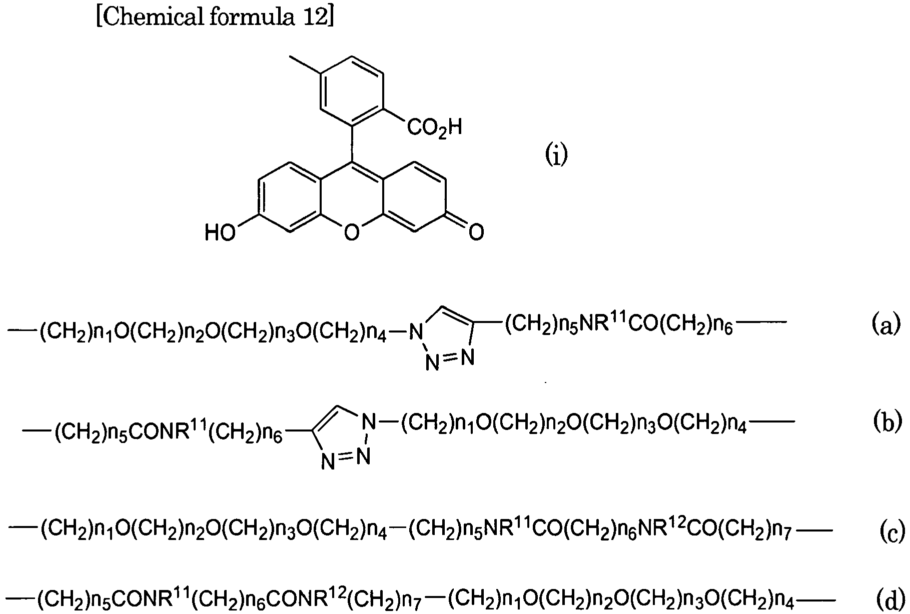

Moreover, the present invention is a compound represented by General Formula (I) or a salt thereof.

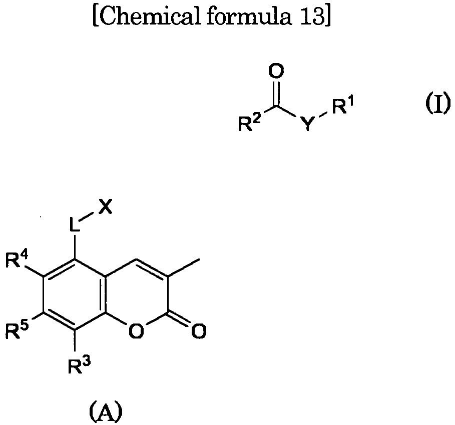

In Formula (I) above,
Y is an oxygen atom or a sulfur atom,
R¹ is a lower alkyl group, a lower alkyl group substituted with halogen, an aryl group, an aryl group substituted with at least one selected from the group consisting of hydroxy, halogen, and lower alkoxy, a lower aralkyl group, or a lower aralkyl group substituted with at least one selected from the group consisting of hydroxy, halogen, and lower alkoxy, and
R² is a group represented by Formula (A) including a coumarin skeleton,
wherein in Formula (A),
X is a fluorescent group,
L is a linker for X,
R³ and R⁴ are each independently a hydrogen atom, a halogen atom, a lower alkyl group, a hydroxy group, a lower alkoxy group, or a lower alkyl group substituted with halogen, and
R⁵ is a hydroxy group, an amino group, an amino group that has been mono- or di-substituted with lower alkyl, or a lower alkoxy group.

Preferably, with respect to the compound of Formula (I), in Formula (I),
Y is a sulfur atom,
R¹ is an aryl group or an aryl group substituted with at least one selected from the group consisting of hydroxy, halogen, and lower alkoxy, and
R² is a group represented by Formula (A),
wherein in Formula (A),
X is a group represented by Formula (i),
L is a group represented by Formula (a), Formula (b), Formula (c), or Formula (d),
R³ and R⁴ each are a hydrogen atom, and
R⁵ is hydroxy,
wherein in Formula (a) and Formula (b),
n1, n2, n3, n4, n5, and n6 are each independently an integer of 0 or 1 to 5, and
R¹¹ is a hydrogen atom or a lower alkyl group, and
in Formula (c) and Formula (d),
n1, n2, n3, n4, n5, n6, and n7 are each independently an integer of 0 or 1 to 5, and
R¹¹ and R¹² are each independently a hydrogen atom or a lower alkyl group.

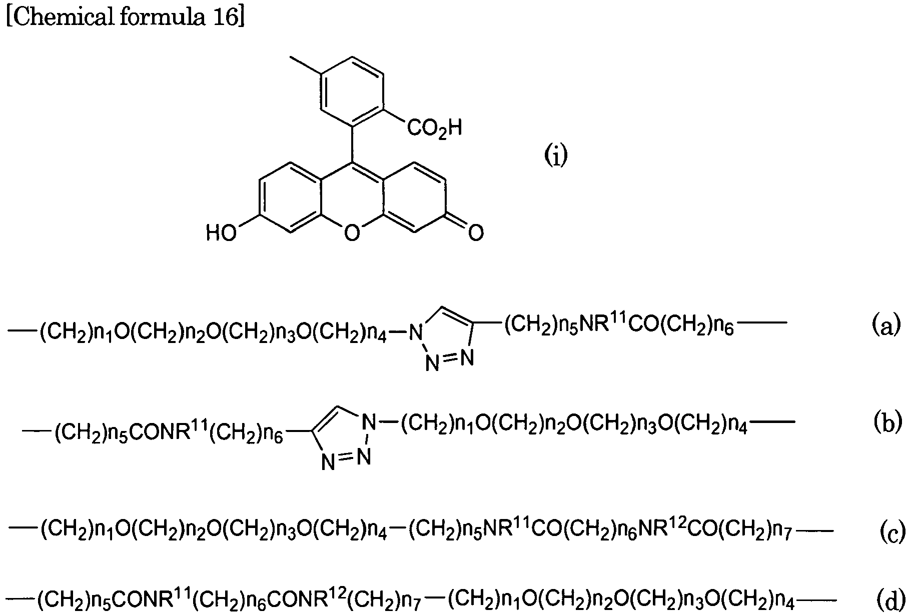

Furthermore, the compound of Formula (I) is more preferably a compound FCTP represented by the following formula. These preferred compounds of Formula (I) are also used preferably in, for example, a method for fluorescently labeling a protein and a composition of the present invention.

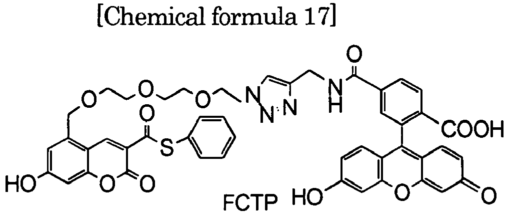

The compound of Formula (I) can be produced, for example, according to Scheme 3 below. Scheme 3 below shows a production example wherein in Formula (I), L is a specific group. The starting material may be produced in house by reference to known documents of conventional techniques or may be purchased.

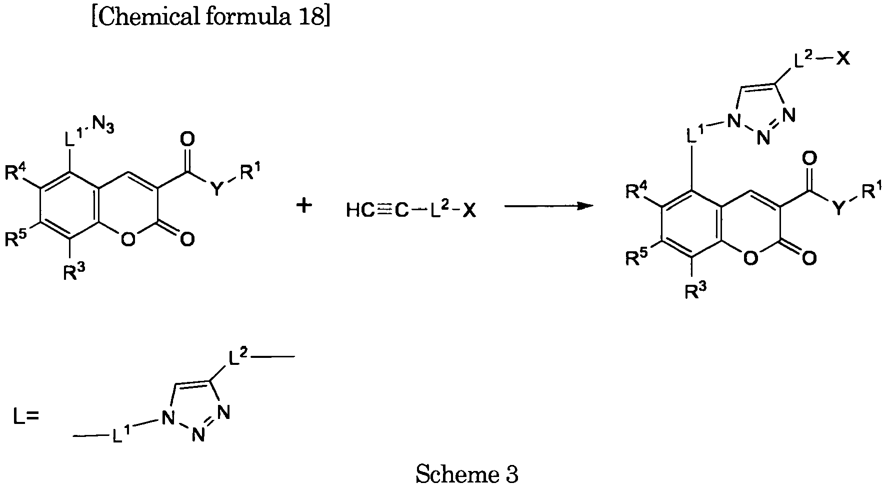

In the above-mentioned formula,
Y is an oxygen atom or a sulfur atom,
R¹ is a lower alkyl group, a lower alkyl group substituted with halogen, an aryl group, an aryl group substituted with at least one selected from the group consisting of hydroxy, halogen, and lower alkoxy, a lower aralkyl group, or a lower aralkyl group substituted with at least one selected from the group consisting of hydroxy, halogen, and lower alkoxy,
X is a fluorescent group,
L is a linker for X,
R³ and R⁴ are each independently a hydrogen atom, a halogen atom, a lower alkyl group, a hydroxy group, a lower alkoxy group, or a lower alkyl group substituted with halogen, and
R⁵ is a hydroxy group, an amino group, an amino group that has been mono- or di-substituted with lower alkyl, or a lower alkoxy group.

The present invention further can provide a bioimaging method that includes labeling a target protein by a method for fluorescently labeling a protein of the present invention. Preferably, the bioimaging method of the present invention includes imaging of a cell or tissue that expresses the target protein. The cell or tissue is not particularly limited but is preferably an animal cell or tissue, more preferably a mammalian cell or tissue, further preferably a primate cell or tissue, and still further preferably a human cell or tissue.

### [Examples]

The following abbreviations are used in the descriptions in this specification.
DMSO: Dimethylsulfoxide
HOBt: 1-hydroxybenzotriazole
DMF: Dimethylformamide
WSCD·HCl: Water-soluble carbodiimide :
1-ethyl-3-(3-dimethylaminopropyl)carbodiimide hydrochloride
MOMCl: Methoxymethylchloride
DIEA: N,N-diisopropylethylamine
NBS: N-bromosuccinimide
AIBN: Azobisbutyronitrile
TFA: Trifluoroacetic acid
TCEP: Tris[2-carboxyethyl]phosphine
SDS-PAGE: Sodium dodecyl sulfate polyacrylamide gel electrophoresis
CBB: Coomassie brilliant blue
DTT: Dithiothreitol
MESNa: 2-mercaptoethanesulfonic acid sodium salt

### [Compounds and Instruments]

Compounds were of the best grade available, purchased from Tokyo Chemical Industry Co., Ltd., Wako Pure Chemical Industries, Ltd., and Sigma-Aldrich Japan, KK, and used without further purification.

NMR spectra were recorded on a JEOL JNM-AL400 instrument (JEOL Ltd.) at 400 MHz for ¹H and at 100.4 MHz for ¹³NMR using tetramethylsilane as an internal standard. Mass spectra (EI) were taken on a Shimazu GCMS-QP2000 (Shimadzu Corporation) operating in the electron impact mode (70 eV) equipped with CBP1-M25-025 column. High-resolution mass spectra (HRMS) were obtained on a JEOL JMS-DX303 (JEOL Ltd.). ESI-TOF MS was taken on a Waters LCT-Premier XE (Nihon Waters KK). Fluorescence spectra were measured using a Hitachi F4500 spectrometer (Hitachi, Ltd.). Slit width was 2.5 nm for both excitation and emission. The photomultiplier voltage was 700 V Samples were dissolved in DMSO (biochemical grade, Wako Pure Chemical Industries, Ltd.) before fluorescence measurement. Silica gel column chromatography was performed using BW-300 (Fuji Silysia Chemical Ltd.). Fluorescence imaging was visualized usingAE-6935B VISIRAYS-B.

### [Example 1]

### Production of Compound FCTP

The following compound FCTP was produced according to Scheme 4 below.

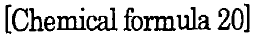

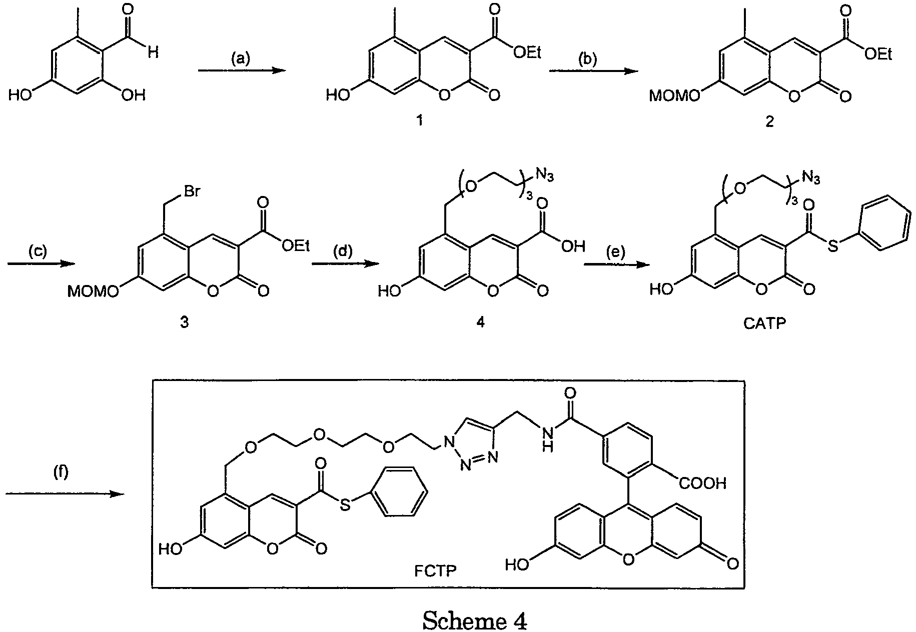

In Scheme 4: (a) diethyl malonate, piperidine/EtOH, (b) MOMCl, DIEA/DMF, (c)NBS, AIBN/CCl₄, (d) 1. 2-[2-(2-azidoethoxy)ethoxy]ethanol, NaH/DMF; 2. 2N aqueous NaOH solution; 3. TFA, (e) thiophenol, WSCD·HCl, HOBt·H₂O/DMF (f) 6-carboxyfluorescein-propargylamide, CuSO₄, sodium ascorbate/DMF/H₂O (4/1).

### Production of 7-hydroxy-5-methylcoumarin-3-carboxylic acid ethyl ester (1)

2,4-dihydroxy-6-methylbenzaldehyde (2.0 g), diethyl malonate (2.2 ml, 14.4 mmol), and piperidine (0.35 ml) were dissolved in ethanol (20 ml), which then was stirred at 100°C for 23 hours. After the mixture was cooled, the crystals obtained thereby were isolated by filtration and washed with cold ethanol (-20°C) to yield compound (1) (2.02 g, yield 62%).
¹H-NMR (400 MHz, CDCl₃): δ1.41 (t, J = 7.2 Hz, 3H), 2.54 (s, 3H), 4.41 (q, J = 7.2 Hz, 2H), 6.71 (d, J = 7.2 Hz, 1H) 6.76 (d, 1H), 8.71 (s, 1H);
¹³C-NMR (100 MHz, CDCl₃):δ 14.1, 17.9, 60.8, 99.9, 109.3, 110.9, 115.2, 140.6, 146.0, 156.4, 157.9, 163.3, 164.2;
HRMS(EI⁺): Calculated 248.06, Found 248.06.

### Production of 7-(methoxymethoxy)-5-methylcoumarin-3-carboxylic acid ethyl ester (2)

7-hydroxy-5-methylcoumarin-3-carboxylic acid ethyl ester (1) (0.20 g, 0.7 mmol), diisopropylethylamine (0.40 ml, 1.8 mmol), and chloromethylmethylether (0.015 ml, 1.8 mmol) were dissolved in DMF, which then was stirred at 0°C for two hours. After the solvent was removed from the mixture, the residue thus obtained was dissolved in a 10% aqueous citric acid solution (30 mL) and extracted with ethyl acetate. The organic phase thus obtained was dried over Na₂SO₄ and the solvent was evaporated to dryness under reduced pressure to yield compound (2) (0.186 mg, yield 79%).
¹H-NMR (400 MHz, CDCl₃); δ 1.41 (t, J = 7.2 Hz, 3H) 2.55 (s, 1H) 3.49 (s, 3H) 4.42 (q, J =7.2 Hz, 2H) 5.23 (s, 2H) 6.82 (d, J = 6.0 Hz, 1H) 6.85 (d, J = 6.0 Hz, 1H) 8.69 (s, 1H);
¹³C-NMR (100 MHz, CDCl₃): δ 14.3, 18.5, 56.5, 61.7, 94.2, 101.2, 111.7, 113.8, 115.3, 139.8, 145.9, 157.1, 157.9, 162.1, 163.8;
HRMS(EI⁺) : Calculated 292.09, Found 292.09.

### Production of 5-bromomethyl-7-(methoxymethoxy)coumarin-3-carboxylic acid ethyl ester (3)

7-(methoxymethoxy)-5-methylcoumarin-3-carboxylic acid ethyl ester (2) (0.5 g, 1.3 mmol), NBS (0.365 g, 2.1 mmol), and AIBN (0.04 g, 0.24 mmol) were dissolved in CCl₄ (15 ml), which then was stirred at 95°C for seven hours. After the solvent was removed from the mixture, the residue thus obtained was eluted with dichloromethane/ethyl acetate (97/3) and purified using silica gel column chromatography to yield compound (3) (0.327 g, yield 52%).
¹H-NMR (400 MHz, CDCl₃); δ 1.42 (t, J = 7.2 Hz, 3H) 3. 50 (s, 3H) 4.42 (q, J = 7.2 Hz, 2H) 4.65 (s, 1H) 5.27 (s, 2H) 6.98 (d, J = 6.0 Hz, 1H) 7.01 (d, J = 6.0 Hz, 1H) 8.77 (s, 1H);
¹³C-NMR(100 MHz, CDCl₃): δ 14.3, 27.9, 56.7, 62.0, 94.5, 103.7, 110.7, 114.8, 115.7, 138.1, 144.5, 156.4, 157.9, 161.7, 163.3;
HRMS(EI⁺) : Calculated 370.01, Found 370.00.

### Production of 5-((2-(2-(2-azidoethoxy)ethoxy)ethoxy)methyl)-7-hydroxycoumarin-3-carboxylic acid (4)

2-[2-(2-azidoethoxy)ethoxy]ethanol (0.233 g, 1.3 mmol) and sodium hydride (0.038 g, 1.6 mmol) were dissolved in DMF (5 ml), which then was stirred at room temperature for 15 minutes. 5-bromomethyl-7-(methoxymethoxy)coumarin-3-carboxylic acid ethyl ester (3) was added to the mixture, and the mixture thus obtained was stirred at room temperature for 1.5 hours. Then, an aqueous NaOH solution (2 N, 3 ml) was added to the mixture. After 20 minutes, the mixture was neutralized with a saturated solution of citric acid and with dichloromethane. After the solvent was removed, the residue thus obtained was dissolved in TFA (10 ml), which then was stirred at room temperature for one hour. After the solvent was removed, the residue obtained by reversed-phase HPLC in which it was eluted with H₂O/acetonitrile containing 0.1% formic acid was purified to yield compound (4) (21 mg, yield 8%).
¹H-NMR (400 MHz, CDCl₃): δ 3.21 (s, 2H) 3.53-3.61 (m, 10H) 6.61 (s, 1H) 6.82 (s, 1H) 8.86 (s, 1H);
MS(ESI⁺) : Calculated 394.35, Found 394.11.

### Production of Compound CATP

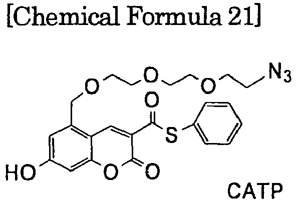

5-((2-(2-(2-azidoethoxy)ethoxy)ethoxy)methyl)-7-hydroxycoumarin-3-carboxylic acid (4) (15.5 mg, 0.039 mmol), WSCD·HCl (9.6 mg, 0.047 mmol), and HOBt·H₂O (7.3 mg, 0.047 mmol) were dissolved in DMF (2 ml), which then was stirred at room temperature for one hour. Thereafter, thiophenol (0.0039 ml, 0.039 mmol) was added to the mixture, which then was stirred for one hour. After the solvent was removed, the residue thus obtained was eluted with dichloromethane/methanol (98/2) and purified by silica gel column chromatography to yield compound CATP (17 mg, 89%).
¹H-NMR (400 MHz, CDCl₃): δ 3.37 (t, J = 4.8 Hz, 2H) 3.60-3.75 (m, 10H) 4.73 (s, 2H) 6.71 (d, J = 2.0 Hz, 1H) 7.01 (d, J = 2.0 Hz, 1H) 7.46 (m, 5H) 8.68 (s, 1H); ¹³C-NMR (100 MHz, CDCl₃): δ 50.5, 69.6, 69.8, 70.1, 70.2, 70.6, 77.0, 70.6, 102.4, 109.6, 114.5, 117.6, 128.3, 129.1, 129.5, 134.9, 140.5, 158.1, 159.2,163.4,187.2;
HRMS (FAB⁺) Calculated 486.14, Found 486.13.

### Production of Compound FCTP

Compound CATP (32 mg, 0.068 mmol), 6-carboxyfluorescein-propargylamide (28 mg, 0.068 mmol), CuSO₄ (8.5 mg, 0.034 mmol), and sodium ascorbate (13.3 mg, 0.068 mmol) were dissolved in DMF/H₂O (4/1), which then was stirred at room temperature for one hour. After the solvent was removed, the residue obtained by reversed-phase HPLC in which it was eluted with 0.1% formic acid-containing H₂O/acetonitrile was purified to yield Compound FCTP (10 mg, yield 16%).
¹H-NMR (400 MHz, DMSO-d₆): δ 3.35-4.35 (m, 8H) 3.70 (t, J = 4.8, 2H) 4.37-4.42 (m, 4H) 4.69 (s, 2H) 6.53-6.69 (m, 7H) 6.80 (s, 1H) 7.46 (s, 5H) 7.71 (s, 1H) 7.86 (s, 1H) 8.05 (d, 1H) 8.18 (d, 1H) 8.66 (s, 1H);
¹³C-NMR (100 MHz, DMSO-d₆): δ 34.9, 49.2, 68.6, 69.2, 69.3, 69.4, 69.5, 69.6, 69.7, 83.4, 101.7,102.2,108.3,108.4,109.1,112.7,115.8,115.9,122.3,122.4,124.8,128.3,128.4, 129.1, 129.2, 129.3, 129.4, 129.5, 134.8, 140.3, 140.9, 144.2, 151.8, 152.7, 158.4, 159.6, 164.4, 168.0, 185.3;
HRMS (FAB⁺) Calculated 899.22, Found 899.23.

### [Cloning of PYP]

With a plasmid (for coding DNA of a His tag fusion protein of a PYP) used as a template, DNA of the His-PYP was amplified by PCR (PrimeSTAR HS DNA Polymerase, TAKARA BIO INC.) using the primers indicated below. The plasmid was provided by Professor K. J. Hellingwerf of University of Amsterdam. The PYP is derived from Ectohiodospira halophila (Halorhodospira halophila). The PCR fragment (insert) was separated with a 2.0% agarose gel and then extracted using a kit (QIA quick Gel Extraction kit, QIAGEN). The vector for E. coli expression, pET21b(+), and the insert were subjected to a restriction enzyme treatment with Hindl III and Nde I (both manufactured by TAKARA BIO INC.), and a T4-DNA ligase was used to integrate the insert into pET-21b(+). Thus, the pET-PYP was produced.
[Chemical Formula 22]
Primers for PCR
Forward Primer (SEQ ID NO: 30):
CGGAGATATACATATGAGAGGATCGCATCAC
Reverse Primer (SEQ ID NO: 31):
GCTAATTAAGCTTCTAGACGC

### [PYP Expression and Purification]

The pETPYP was transformed into E. coli (BL21 (DE3)) and then grown to an OD600 of 0.6 to 0.8 Å in Luria-Bertani (LB) medium at 37 °C. Next, IPTG was added with a final concentration of 100 µM and this was grown overnight at 20 °C. The E. coli thus grown was harvested and then was subjected to ultrasonic fragmentation. Thereafter, a soluble fraction was extracted. The soluble fraction PYP was purified with the Ni-NTA column (Ni-NTA agarose; QIAGEN) using the buffers described below according to the QIAGEN'S protocol. Then the fraction was subjected to final purification by gel filtration column chromatography (SuperdexTM 75 10/300 GL, GE Healthcare) using Tris buffer (20 mM Tris-HCl, 150 mM NaCl, 1 mM TCEP, pH 8.0) as a mobile phase.

Conditions for Ni-NTA column include:
a binding solution: phosphoric acid buffer (50 mM sodium phosphate, 300 mM NaCl, 10 mM imidazole, pH 8.0),
a washing solution: phosphoric acid buffer (50 mM sodium phosphate, 300 mM NaCl, 20 mM imidazole, pH 8.0), and
an eluate: phosphoric acid buffer (50 mM sodium phosphate, 300 mM NaCl, 50 mM imidazole, pH 8.0).

### [Treatment of PYP with Enterokinase]

A sequence to be cleaved by a protease, enterokinase, has been inserted between His tag and PYP In order to remove the His tag, enterokinase (Recombinant Enterokinase; Novagen) was added to the purified PYP, which then was allowed to stand at room temperature for 18 hours. Thereafter, a sample was added to the Ni-NTA column (Ni-NTA agarose; QIAGEN) and the His tag site was adsorbed thereto. Thus, cleaved PYP was purified. The sample from which the His tag site had been removed was added to a benzamidine column (HiTrap Benzamidine FF, GE Healthcare) and thereby protease was removed. The purified sample was subjected to ultrafiltration (VIVASPIN 500, 5000 MWCO PES, Sartorius Stedim) to substitute Tris buffer (20 mM Tris-HCl, 150 mM NaCl, 1 mM TCEP, pH 8.0).

### [Reaction between PYP and FCTP]

In Tris buffer (20 mM Tris-HCl, 150 mM NaCl, 1 mM TCEP (pH 8.0)), 5 µM of PYP was reacted with 12.5 µM of compound FCTP at 37°C for 24 hours. After completion of the reaction, the result was analyzed by SDS-PAGE (20% acrylamide separation gel). The resultant CBB-stained image is shown in FIG. 1(a) and the resultant fluorescent image is shown in FIG. 1(b). With reference to FIG. 1(a), in the CBB-stained image, a slowly migrating band was observed with the addition of compound FCTP. With reference to FIG. 1(b), similarly in the fluorescent image, fluorescence was observed. Thus, it was confirmed that compound FCTP was able to label the PYP Furthermore, since an addition of DTT (100 mM) reduced the slowly migrating band and also made the fluorescence intensity weaker, it was confirmed that the thioester bond between the PYP and compound CATP was hydrolyzed by a large excess of DTT

### [Reaction of Compound FCTP with PYP in Cell Lysate]

In a HEK293T cell lysate dissolved in Tris buffer (20 mM Tris-HCl, 150 mM NaCl, 1 mM TCEP (pH 8.0)), 5 µM of PYP was reacted with 50 µM of compound FCTP at 37°C for 30 minutes in the presence or absence of 10 mM MESNa. After completion of the reaction, it was analyzed by SDS-PAGE (20% acrylamide separation gel). The resultant CBB-stained image is shown in FIG. 2(a) and the resultant fluorescent image is shown in FIG. 2(b). As shown in FIGS. 2(a) and 2(b), with an addition of compound FCTP, fluorescence was observed in the vicinity of the PYP band confirmed by the CBB-stained image. Furthermore, another fluorescent band was observed in the high molecular weight range. On the other hand, the fluorescent band on the high molecular weight side disappeared upon addition of MESNa (10 mM), which was a type of thiol compound. It was confirmed that an addition of a certain amount of free thiol allowed labeling to be carried out specifically in the cell lysate.

### [Fluorescent properties of FCTP]

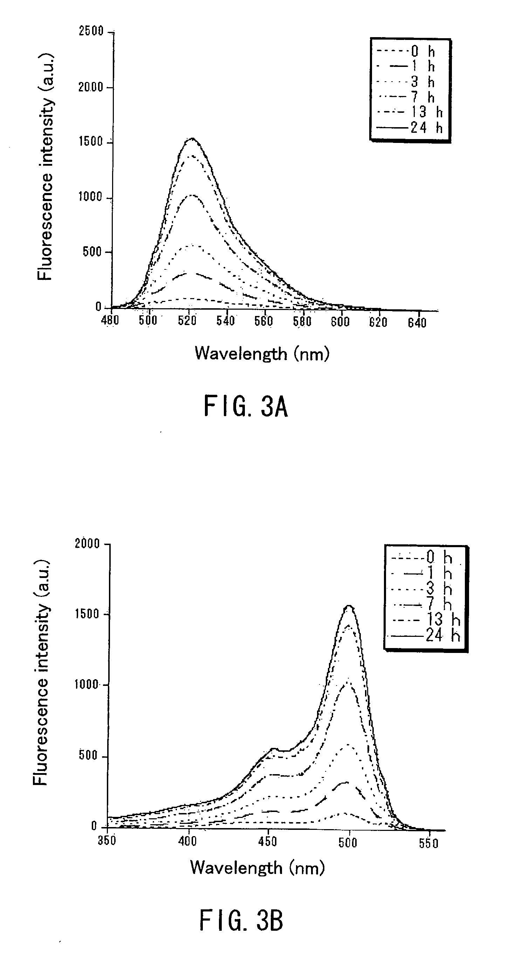

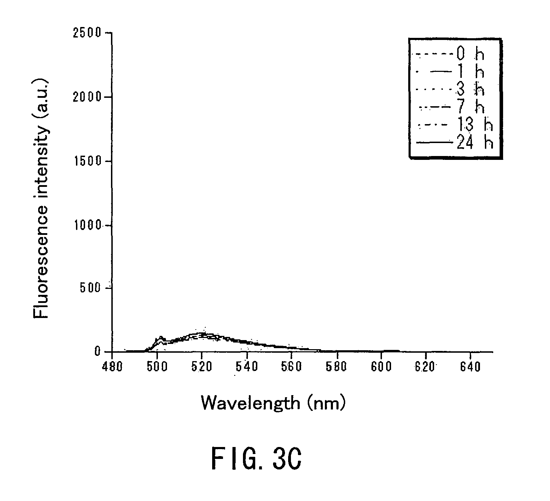

In order to examine the fluorescent properties of compound FCTP bound to the PYP, Tris buffer (20 mM Tris-HCl, 150 mM NaCl, 1 mM TCEP (pH 8.0)), the fluorescence and excitation spectra were measured at 37°C. FIG. 3(a) shows time-dependent fluorescence spectra of 8 µM of compound FCTP in the presence of 5 µM of PYP, FIG. 3(b) shows time-dependent excitation spectra of 8 µM of compound FCTP in the presence of 5 µM of PYP, and FIG. 3(c) shows fluorescence spectra (excitation at 500 nm) of 8 µM of compound FCTP.

As shown in FIG. 3(c), in the case of compound FCTP alone, the fluorescence intensity was very weak and no change in fluorescence spectra was observed with the passage of time. On the other hand, as shown in FIGS. 3(a) and 3(b), the fluorescence intensity of compound FCTP increased in the presence of the PYP with the passage of time and changes in both fluorescence and excitation spectra were observed. From these results, it was confirmed that compound FCTP had a weaker fluorescence intensity before binding to the PYP and the fluorescence intensity increased considerably upon binding.

### [Labeling Reaction with FCTP in Cultured Cell]

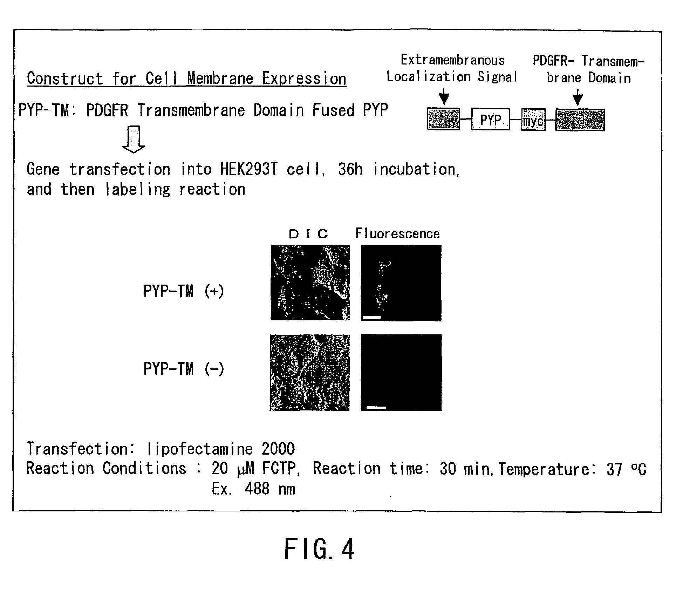

In order to express a PYP on the cell surface, a plasmid was prepared in which a gene PYP-TM obtained by fusing a PYP with the transmembrane domain of a platelet-derived growth factor receptor (PDGFR) was introduced into the multicloning site of pcDNA3.1, which is a mammalian expression vector. HEK293T cells were transfected with this plasmid by using lipofectamine 2000. After transfection with the plasmid, the HEK293T cells were allowed to stand at 37°C for 36 hours and then washed with a phosphoric acid buffer (PBS). A culture medium containing FCTP (20 µM) was added thereto, which then was allowed to stand for 30 minutes. Thereafter, the culture media was replaced by a culture medium containing 10% fetal bovine serum (FBS) and the cells then were observed with a fluorescence microscope. With the fluorescence microscope having an excitation wavelength of 488 nm, the cells were observed using a fluorescent filter of 510 to 550 nm. The microscopic images obtained thereby are shown in FIG. 4.

### [Labeling Reaction with CATP in Cultured Cell]

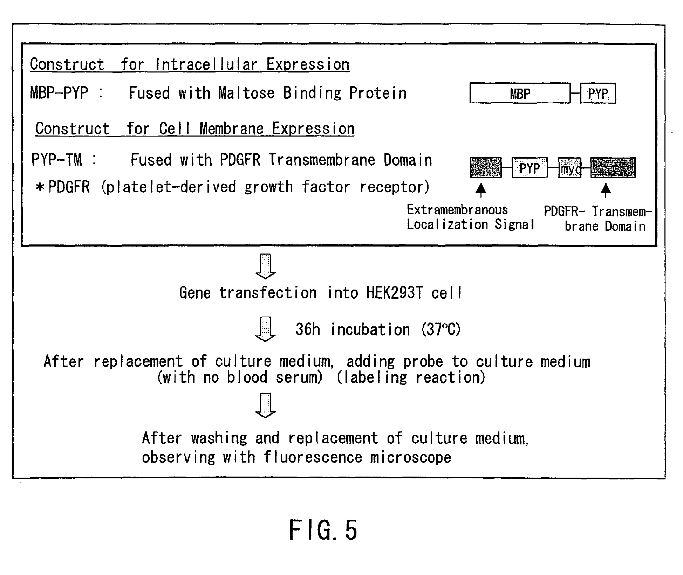

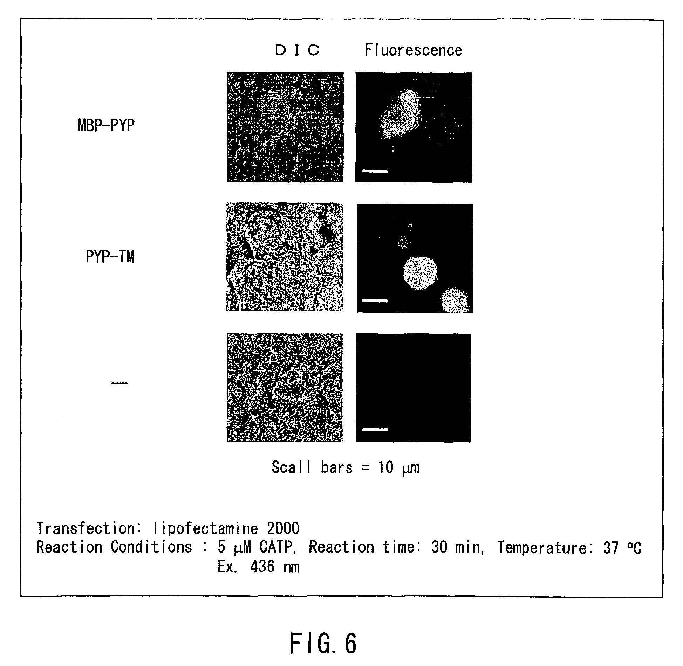

A plasmid was prepared in which a gene MBP-PYP obtained by fusing a maltose binding protein (MBP) with a PYP was introduced into the multicloning site of pcDNA3.1, which was a mammalian expression vector. Furthermore, in order to express a PYP on the cell surface, a plasmid was prepared in which a gene PYP-TM obtained by fusing a PYP with the transmembrane domain of a platelet-derived growth factor receptor (PDGFR) was introduced into the multicloning site of pcDNA3.1, which was a mammalian expression vector. HEK293T cells were transfected with these plasmids by using lipofectamine 2000. After transfection with the plasmids, the HEK293T cells were allowed to stand at 37°C for 36 hours and then washed with a phosphoric acid buffer (PBS). A culture medium containing CATP (5 µM) was added thereto, which then was allowed to stand for 30 minutes. Thereafter, the culture medium was replaced by a culture medium containing 10% fetal bovine serum (FBS) and the cells then were observed with a fluorescence microscope. With the fluorescence microscope having an excitation wavelength of 436 nm, the cells were observed with a fluorescent filter of 470 to 495 nm. FIG. 5 shows the reaction procedure and FIG. 6 shows the fluorescence microscopic images obtained thereby.

As shown in FIGS. 4 and 6, both fluorescent labeling with FCTP and that with CATP were observed in the PYP-TM expressed cells. On the other hand, neither fluorescent labeling with FCTP nor that with CATP was observed in the cells where PYP-TM was not expressed. These results confirmed that both FCTP and CATP specifically labeled the PYP on the cell membranes. Furthermore, as shown in FIG. 6, fluorescent labeling with CATP was observed in the cells in which MBP-PYP was expressed, while fluorescent labeling with CATP was not observed in the cells in which MBP-PYP was not expressed. These results confirmed that CATP specifically labeled the PYP inside the cultured cell.

### Industrial Applicability

The method for fluorescently labeling a protein of the present invention can be used, for example, for cell imaging.

### [Sequence Listing Free Text]

SEQ ID NO: 30: Forward Primer
SEQ ID NO: 31: Reverse Primer

## Claims

1. A method for fluorescently labeling a protein comprising:
obtaining a fusion protein of a target protein to be labeled and a photoactive yellow protein (PYP) or a PYP-derived protein, and fluorescently labeling the target protein by reacting the fusion protein with a compound represented by Formula (I) below or a salt thereof,
wherein in the compound represented by Formula (I) or the salt thereof, a coumarin skeleton in Formula (A) is intramolecularly associated with a group X and as a result, fluorescence derived from the group X is suppressed, and
when in the compound represented by Formula (I) or the salt thereof, the coumarin skeleton in Formula (A) is bound to the PYP or the PYP-derived protein contained in the fusion protein, the coumarin skeleton in Formula (A) is intramolecularly dissociated from the group X, and thereby fluorescence derived from the group X can be emitted, 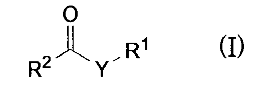 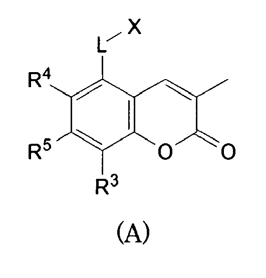 wherein in Formula (I),
Y is an oxygen atom or a sulfur atom,
R¹ is a lower alkyl group, a lower alkyl group substituted with halogen, an aryl group, an aryl group substituted with at least one selected from the group consisting of hydroxy, halogen, and lower alkoxy, a lower aralkyl group, or a lower aralkyl group substituted with at least one selected from the group consisting of hydroxy, halogen, and lower alkoxy, and
R² is a group represented by Formula (A) including the coumarin skeleton,
wherein in Formula (A),
X is a fluorescent group,
L is a linker for X,
R³ and R⁴ are each independently a hydrogen atom, a halogen atom, a lower alkyl group, a hydroxy group, a lower alkoxy group, or a lower alkyl group substituted with halogen, and
R⁵ is a hydroxy group, an amino group, an amino group that has been mono- or di-substituted with lower alkyl, or a lower alkoxy group, and
wherein the amino acid sequence of the PYP or PYP-derived protein is one selected from the group consisting of.
1) amino acid sequences represented by SEQ ID NOS: 1 to 17 of the sequence listing,
2) amino acid sequences represented by SEQ ID NOS: 1 to 17 of the sequence listing in which the 1st to 24th, 25th, 26th, or 27th amino acids have been deleted,
3) amino acid sequences in which one to several amino acids of the amino acid sequences of either 1) or 2) described above have been deleted, substituted, or added, wherein when the fusion protein binds to the coumarin skeleton in Formula (A), the coumarin skeleton in Formula (A) is intramolecularly dissociated from the group X in the compound represented by Formula (I), and thereby fluorescence derived from the group X can be emitted, and
4) amino acid sequences having a homology of at least 70% with the amino acid sequences of either 1) or 2) described above, wherein when the fusion protein binds to the coumarin skeleton in Formula (A), the coumarin skeleton in Formula (A) is intramolecularly dissociated from the group X in the compound represented by Formula (I), and thereby fluorescence derived from the group X can be emitted.

2. The method for fluorescently labeling a protein according to claim 1, wherein obtaining the fusion protein comprises obtaining a polynucleotide for coding the fusion protein, obtaining a plasmid or vector that can express the fusion protein, expressing the fusion protein in a cell, or isolating the fusion protein thus expressed.

3. A compound represented by General Formula (I) or a salt thereof, 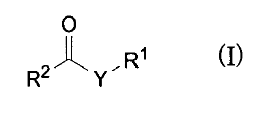 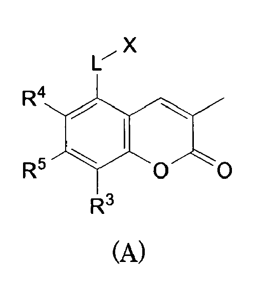 wherein in Formula (I),
Y is an oxygen atom or a sulfur atom,
R¹ is a lower alkyl group, a lower alkyl group substituted with halogen, an aryl group, an aryl group substituted with at least one selected from the group consisting of hydroxy, halogen, and lower alkoxy, a lower aralkyl group, or a lower aralkyl group substituted with at least one selected from the group consisting of hydroxy, halogen, and lower alkoxy, and
R² is a group represented by Formula (A) including a coumarin skeleton,
wherein in Formula (A),
X is a fluorescent group,
L is a linker for X,
R³ and R⁴ are each independently a hydrogen atom, a halogen atom, a lower alkyl group, a hydroxy group, a lower alkoxy group, or a lower alkyl group substituted with halogen, and
R⁵ is a hydroxy group, an amino group, an amino group that has been mono- or di-substituted with lower alkyl, or a lower alkoxy group.

4. The compound or the salt thereof according to claim 3, wherein
Y is a sulfur atom,
R¹ is an aryl group or an aryl group substituted with at least one selected from the group consisting of hydroxy, halogen, and lower alkoxy, and
R² is a group represented by Formula (A),
wherein in Formula (A),
X is a group represented by Formula (i),
L is a group represented by Formula (a), Formula (b), Formula (c), or Formula (d), R³ and R⁴ each are a hydrogen atom, and
R⁵ is hydroxy,
wherein in Formula (a) and Formula (b),
n1, n2, n3, n4, n5, and n6 are each independently an integer of 0 or 1 to 5, and
R¹¹ is a hydrogen atom or a lower alkyl group, and
in Formula (c) and Formula (d),
n1, n2, n3, n4, n5, n6, and n7 are each independently an integer of 0 or 1 to 5, and R¹¹ and R¹² are each independently a hydrogen atom or a lower alkyl group, 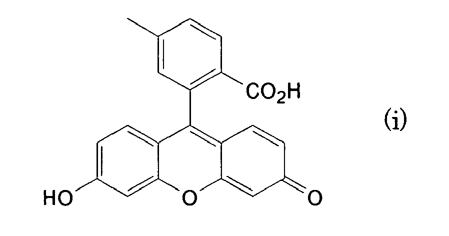 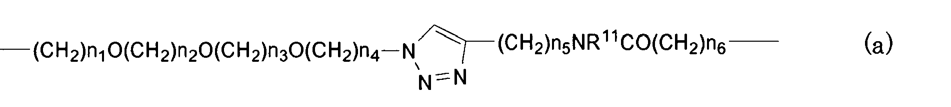 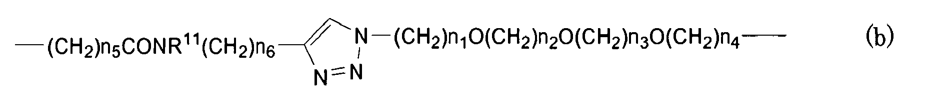
-(CH₂)n₁O(CH₂)n₂O(CH₂)n₃O(CH₂)n₄-(CH₂)n₅NR¹¹CO(CH₂)n₆NR¹²CO(CH₂)n₇- (c)
-(CH₂)n₅CONR¹¹(CH₂)n₆CONR¹²(CH₂)n₇-(CH₂)n₁O(CH₂)n₂O(CH₂)n₃O(CH₂)n₄- (d)

5. A composition to be used for a method according to claim 1 or claim 2, comprising a compound or a salt thereof according to claim 3 or claim 4.

6. A kit for a method for fluorescently labeling a protein, comprising a composition according to claim 5 and a plasmid or vector,
wherein the plasmid or vector is one for cloning a polynucleotide for coding a fusion protein of a target protein to be labeled and a PYP or PYP-derived protein as defined in claim 1 or one for expressing the fusion protein,
the plasmid or vector comprises a base sequence selected from the group consisting of:
1) base sequences represented by SEQ ID NOS: 18 to 29 of the sequence listing,
2) base sequences represented by SEQ ID NOS: 18 to 29 of the sequence listing in which base sequences have been deleted corresponding to deletion of the 1st to 24th, 25th, 26th, or 27th at the N terminus of the amino acid sequences that are coded with the base sequences,
3) base sequences in which one to several bases of the base sequences of either 1) or 2) described above have been deleted, substituted, or added, wherein the base sequences code amino acid sequences in which when the fusion protein binds to the coumarin skeleton in Formula (A), the coumarin skeleton in Formula (A) is intramolecularly dissociated from the group X in the compound represented by Formula (I), and thereby fluorescence derived from the group X can be emitted, and
4) base sequences that have a homology of at least 70% with the base sequences of either 1) or 2) described above, wherein the base sequences code amino acid sequences in which when the fusion protein binds to the coumarin skeleton in Formula (A) below, the coumarin skeleton in Formula (A) is intramolecularly dissociated from the group X in the compound represented by Formula (I), and thereby fluorescence derived from the group X can be emitted, 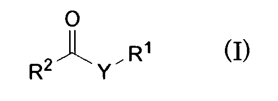 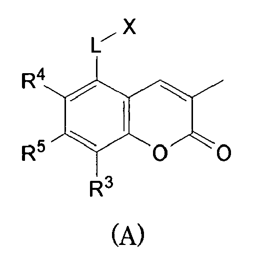 wherein in Formula (I),
Y is an oxygen atom or a sulfur atom,
R¹ is a lower alkyl group, a lower alkyl group substituted with halogen, an aryl group, an aryl group substituted with at least one selected from the group consisting of hydroxy, halogen, and lower alkoxy, a lower aralkyl group, or a lower aralkyl group substituted with at least one selected from the group consisting of hydroxy, halogen, and lower alkoxy, and
R² is a group represented by Formula (A) including the coumarin skeleton,
wherein in Formula (A),
X is a fluorescent group,
L is a linker for X,
R³ and R⁴ are each independently a hydrogen atom, a halogen atom, a lower alkyl group, a hydroxy group, a lower alkoxy group, or a lower alkyl group substituted with halogen, and
R⁵ is a hydroxy group, an amino group, an amino group that has been mono- or di-substituted with lower alkyl, or a lower alkoxy group.

## Patentansprüche

1. Verfahren zum Fluoreszenz-Markieren eines Proteins, das Folgendes umfasst:
Erhalten eines Fusionsproteins aus einem zu markierenden Zielprotein und einem photoaktiven Yellow-Protein (PYP) oder einem von PYP abgeleiteten Protein und Fluoreszenz-Markieren des Zielproteins durch Umsetzen des Fusionsproteins mit einer durch untenstehende Formel (I) dargestellten Verbindung oder einem Salz davon,
wobei in der durch Formel (I) dargestellten Verbindung oder dem Salz davon ein Cumarinskelett in Formel (A) intramolekular mit einer Gruppe X verbunden ist und als Folge davon die von der Gruppe X stammende Fluoreszenz unterdrückt ist und
wenn in der durch Formel (I) dargestellten Verbindung oder dem Salz davon das Cumarinskelett in Formel (A) an das in dem Fusionsprotein enthaltene PYP oder das von PYP abgeleitete Protein gebunden wird, wird das Cumarinskelett in Formel (A) von der Gruppe X intramolekular dissoziiert und dadurch kann von der Gruppe X stammende Fluoreszenz abgestrahlt werden, 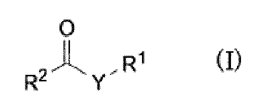 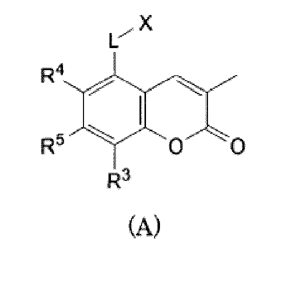
wobei in Formel (I),
Y ein Sauerstoffatom oder ein Schwefelatom ist,
R^{1 1} eine niedere Alkylgruppe ist, eine mit Halogen substituierte niedere Alkylgruppe, eine Arylgruppe, eine Arylgruppe, die mit mindestens einem Substituenten ausgewählt aus der Gruppe bestehend aus Hydroxy, Halogen und niederem Alkoxy substituiert wird, eine niedere Aralkylgruppe oder eine niedere Aralkylgruppe, die mit mindestens einem Substituenten ausgewählt aus der Gruppe bestehend aus Hydroxy, Halogen und niederem Alkoxy substituiert wird und
R² eine Gruppe ist, die durch Formel (A) dargestellt wird einschließlich des Cumarinskeletts,
wobei in Formel (A)
X eine fluoreszierende Gruppe ist,
L ein Verbindungsstück für X ist,
R³ und R⁴ unabhängig voneinander ein Wasserstoffatom, ein Halogenatom, eine niedere Alkylgruppe, eine Hydroxygruppe, eine niedere Alkoxygruppe oder eine niedere Alkylgruppe darstellen, die mit Halogen substituiert wird, und
R⁵ eine Hydroxygruppe ist, eine Aminogruppe, eine Aminogruppe, die mit einer niederen Alkyl- oder einer niederen Alkoxygruppe mono- oder disubstituiert wurde und
wobei die Aminosäuresequenz des PYP oder von PYP abgeleiteten Proteins ausgewählt wird aus der Gruppe bestehend aus:
1) Aminosäuresequenzen, die durch SEQ ID NR: 1 bis 17 des Sequenzprotokolls dargestellt werden,
2) Aminosäuresequenzen, die durch SEQ ID NR: 1 bis 17 des Sequenzprotokolls dargestellt werden, bei denen die 1. bis 24., 25., 26. oder 27. Aminosäuren deletiert wurden,
3) Aminosäuren, bei denen ein bis mehrere Aminosäuren der oben beschriebenen Aminosäuresequenzen 1) oder 2) deletiert, substituiert oder hinzugefügt wurden, wobei wenn das Fusionsprotein an das Cumarinskelett in Formel (A) gebunden wird, das Cumarinskelett in Formel (A) von der Gruppe X in der durch Formel (I) dargestellten Verbindung intramolekular dissoziiert wird und dadurch von der Gruppe X stammende Fluoreszenz abgestrahlt werden kann und
4) Aminosäuresequenzen, die eine Homologie von mindestens 70% mit den oben beschriebenen Aminosäuresequenzen von entweder 1) oder 2) aufweisen, wobei wenn das Fusionsprotein an das untenstehende Cumarinskelett in Formel (A) gebunden wird, das Cumarinskelett in Formel (A) von der Gruppe X in der durch Formel (I) dargestellten Verbindung intramolekular dissoziiert wird und dadurch von der Gruppe X stammende Fluoreszenz abgestrahlt werden kann.

2. Verfahren zum Fluoreszenz-Markieren eines Proteins nach Anspruch 1, wobei das Erhalten des Fusionsproteins das Erhalten eines Polynukleotids umfasst, das für das Fusionsprotein kodiert, das Erhalten eines Plasmids oder eines Vektors, der das Fusionsprotein exprimieren kann, das Exprimieren des Fusionsproteins in einer Zelle oder das Isolieren des derart exprimierten Fusionsproteins.

3. Verbindung, die durch die allgemeine Formel (I) dargestellt wird oder ein Salz davon, 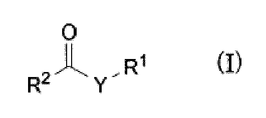 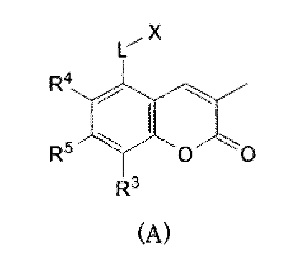 wobei in Formel (I)
Y ein Sauerstoffatom oder ein Schwefelatom ist,
R¹ eine niedere Alkylgruppe ist, eine mit Halogen substituierte niedere Alkylgruppe, eine Arylgruppe, eine Arylgruppe, die mit mindestens einem Substituenten ausgewählt aus der Gruppe bestehend aus Hydroxy, Halogen und niederem Alkoxy substituiert wird, eine niedere Aralkylgruppe oder eine niedere Aralkylgruppe, die mit mindestens einem Substituenten ausgewählt aus der Gruppe bestehend aus Hydroxy, Halogen und niederem Alkoxy substituiert wird und
R² eine Gruppe ist, die durch Formel (A) dargestellt wird einschließlich eines Cumarinskeletts,
wobei in Formel (A)
X eine fluoreszierende Gruppe ist,
L ein Verbindungsstück für X ist,
R³ und R⁴ unabhängig voneinander ein Wasserstoffatom, ein Halogenatom, eine niedere Alkylgruppe, eine Hydroxygruppe, eine niedere Alkoxygruppe oder eine niedere Alkylgruppe darstellen, die mit Halogen substituiert wird, und
R⁵ eine Hydroxygruppe ist, eine Aminogruppe, eine Aminogruppe, die mit einer niederen Alkyl- oder einer niederen Alkoxygruppe mono- oder disubstituiert wurde.

4. Verbindung oder ein Salz davon nach Anspruch 3, wobei
Y ein Schwefelatom ist,
R¹ eine Arylgruppe ist oder eine Arylgruppe, die mit mindestens einem Substituenten ausgewählt aus der Gruppe bestehend aus Hydroxy, Halogen und niederem Alkoxy substituiert wird, und
R² eine Gruppe ist, die durch Formel (A) dargestellt wird,
wobei in Formel (A)
X eine Gruppe ist, die durch Formel (i) dargestellt wird,
L eine Gruppe ist, die durch Formel (a), Formel (b), Formel (c) oder Formel (d) dargestellt wird,
R³ und R⁴ jeweils ein Wasserstoffatom darstellen, und
R⁵ eine Hydroxygruppe ist,
wobei in Formel (a) und Formel (b)
n1, n2, n3, n4, n5 und n6 jeweils unabhängig voneinander ein ganze Zahl von 0 oder 1 bis 5 darstellen und R¹¹ ein Wasserstoffatom oder eine niedere Alkylgruppe darstellt und
in Formel (c) und Formel (d)
n1, n2, n3, n4, n5, n6 und n7 jeweils unabhängig voneinander ein ganze Zahl von 0 oder 1 bis 5 darstellen und R¹¹ und R¹² unabhängig voneinander ein Wasserstoffatom oder eine niedere Alkylgruppe darstellen, 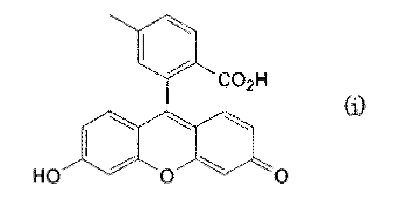 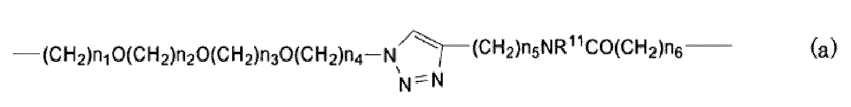 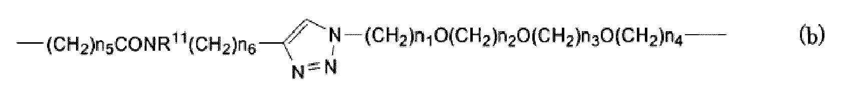
-(CH₂)n₁O(CH₂)n₂O(CH₂)n₃O(CH₂)n₄-(CH₂)n₅NR¹¹CO(CH₂)n₆NR¹²CO(CH₂)n₇- (c)
-(CH₂)n₅CONR¹¹(CH₂)n₆CONR¹²(CH₂)n₇-(CH₂)n₁O(CH₂)n₂O(CH₂)n₃O(CH₂)n₄- (d)

5. Zusammensetzung zur Verwendung in einem Verfahren nach Anspruch 1 oder Anspruch 2, die eine Verbindung oder ein Salz davon nach Anspruch 3 oder Anspruch 4 umfasst.

6. Kit für ein Verfahren zum Fluoreszenz-Markieren eines Proteins, der eine Zusammensetzung nach Anspruch 5 und ein Plasmid oder einen Vektor umfasst,
wobei das Plasmid oder der Vektor zum Klonieren eines Polynukleotids sind, zum Kodieren für ein Fusionsprotein eines zu markierenden Zielproteins und eines PYP oder von PYP abgeleiteten Proteins wie in Anspruch 1 definiert, oder zum Exprimieren des Fusionsproteins,
das Plasmid oder der Vektor eine Basensequenz umfassen, die ausgewählt ist aus der Gruppe bestehend aus:
1) Basensequenzen dargestellt durch SEQ ID NR: 18 bis 29 des Sequenzprotokolls,
2) Basensequenzen dargestellt durch SEQ ID NR: 18 bis 29 des Sequenzprotokolls, bei denen Basensequenzen deletiert wurden entsprechend der Deletion der ersten 1. bis 24., 25., 26. oder 27. am N-terminalen Ende der Aminosäuresequenzen, die von den Basensequenzen kodiert werden,
3) Basensequenzen, bei denen eine bis mehrere Basen der oben beschriebenen Basensequenzen von entweder 1) oder 2) deletiert, substituiert oder hinzugefügt wurden, wobei die Basensequenzen für Aminosäuresequenzen kodieren, bei denen, wenn das Fusionsprotein an das Cumarinskelett in Formel (A) gebunden wird, das Cumarinskelett in Formel (A) von der Gruppe X in der durch Formel (I) dargestellten Verbindung intramolekular dissoziiert wird und dadurch von der Gruppe X stammende Fluoreszenz abgestrahlt werden kann, sowie
4) Basensequenzen, die eine Homologie von mindestens 70% mit der oben beschriebenen Basensequenz von entweder 1) oder 2) aufweisen, wobei die Basensequenzen für Aminosäuresequenzen kodieren, bei denen, wenn das Fusionsprotein an das untenstehende Cumarinskelett in Formel (A) gebunden wird, das Cumarinskelett in Formel (A) von der Gruppe X in der durch Formel (I) dargestellten Verbindung intramolekular dissoziiert wird und dadurch von der Gruppe X stammende Fluoreszenz abgestrahlt werden kann, 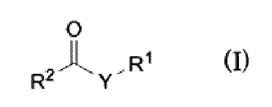 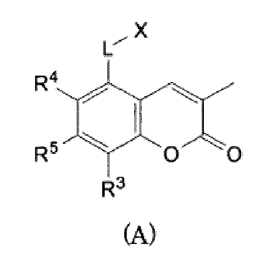 wobei in Formel (I)
Y ein Sauerstoffatom oder ein Schwefelatom ist,
R¹ eine niedere Alkylgruppe ist, eine mit Halogen substituierte niedere Alkylgruppe, eine Arylgruppe, eine Arylgruppe, die mit mindestens einem Substituenten ausgewählt aus der Gruppe bestehend aus Hydroxy, Halogen und niederem Alkoxy substituiert wird, eine niedere Aralkylgruppe oder eine niedere Aralkylgruppe, die mit mindestens einem Substituenten ausgewählt aus der Gruppe bestehend aus Hydroxy, Halogen und niederem Alkoxy substituiert wird und
R² eine Gruppe ist, die durch Formel (A) dargestellt wird einschließlich des Cumarinskeletts,
wobei in Formel (A)
X eine fluoreszierende Gruppe ist,
L ein Verbindungsstück für X ist,
R³ und R⁴ unabhängig voneinander ein Wasserstoffatom, ein Halogenatom, eine niedere Alkylgruppe, eine Hydroxygruppe, eine niedere Alkoxygruppe oder eine niedere Alkylgruppe darstellen, die mit Halogen substituiert wird, und
R⁵ eine Hydroxygruppe ist, eine Aminogruppe, eine Aminogruppe, die mit einer niederen Alkyl- oder einer niederen Alkoxygruppe mono- oder disubstituiert wurde.

## Revendications

1. Procédé de marquage par fluorescence d'une protéine comprenant :
l'obtention d'une protéine de fusion d'une protéine cible à marquer et d'une protéine jaune photoactive (PJP) ou d'une protéine dérivée d'une PJP et le marquage par fluorescence de la protéine cible par réaction de la protéine de fusion avec un composé représenté par la formule (I) ci-dessous ou avec un sel de celui-ci,
dans lequel, dans le composé représenté par la formule (I) ou dans le sel de celui-ci, une chaîne principale de coumarine dans la formule (A) est associée par voie intramoléculaire à un groupe X et il en résulte que la fluorescence dérivée du groupe X est empêchée, et
lorsque, dans le composé représenté par la formule (I) ou dans le sel de celui-ci, la chaîne principale de coumarine dans la formule (A) est liée à la PJP ou à la protéine dérivée d'une PJP contenue dans la protéine de fusion, la chaîne principale de coumarine dans la formule (A) est dissociée sur le plan intramoléculaire du groupe X et de ce fait une fluorescence dérivée du groupe X peut être émise, 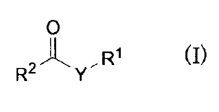 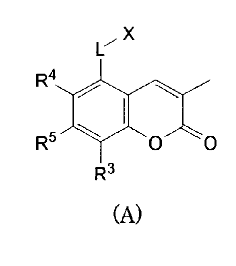 dans lequel, dans la formule (I),
Y est un atome d'oxygène ou un atome de soufre,
R¹ est un groupe alkyle inférieur, un groupe alkyle inférieur substitué par un atome d'halogène, un groupe aryle, un groupe aryle substitué par au moins un substituant choisi dans le groupe constitué d'un groupe hydroxy, d'un atome d'halogène et d'un groupe alcoxy inférieur, un groupe aralkyle inférieur ou un groupe aralkyle inférieur substitué par au moins un substituant choisi dans le groupe constitué d'un groupe hydroxy,
d'un atome d'halogène et d'un groupe alcoxy inférieur, et
R² est un groupe représenté par la formule (A) comprenant la chaîne principale de coumarine,
dans lequel, dans la formule (A),
X est un groupe fluorescent,
L est un segment de liaison pour X,
R³ et R⁴ sont chacun indépendamment un atome d'hydrogène, un atome d'halogène, un groupe alkyle inférieur, un groupe hydroxy, un groupe alcoxy inférieur ou un groupe alkyle inférieur substitué par un atome d'halogène, et
R⁵ est un groupe hydroxy, un groupe amino, un groupe amino qui a été monosubstitué ou disubstitué par un groupe alkyle inférieur ou un groupe alcoxy inférieur, et
dans lequel la séquence d'acides aminés de la PJP ou de la protéine dérivée d'une PJP est une séquence choisie dans le groupe constitué :
1) des séquences d'acides aminés représentées par les SEQ ID N° : 1 à 17 de la liste de séquences,
2) des séquences d'acides aminés représentées par les SEQ ID N° : 1 à 17 de la liste de séquences dans laquelle les 1^{er} au 24^{ème}, 25^{ème}, 26^{ème} ou 27^{ème} acides aminés ont été supprimés,
3) des séquences d'acides aminés dans lesquelles un à plusieurs acides aminés des séquences d'acides aminés de 1) ou de 2) décrites ci-dessus ont été supprimés, substitués ou ajoutés, dans lequel, lorsque la protéine de fusion se lie à la chaîne principale de coumarine dans la formule (A), la chaîne principale de coumarine dans la formule (A) est dissociée sur le plan intramoléculaire du groupe X dans le composé représenté par la formule (I) et, de ce fait, une fluorescence dérivée du groupe X peut être émise, et
4) des séquences d'acides aminés ayant une homologie d'au moins 70 % avec les séquences d'acides aminés de 1) ou de 2) décrites ci-dessus, dans lequel, lorsque la protéine de fusion se lie à la chaîne principale de coumarine dans la formule (A), la chaîne principale de coumarine dans la formule (A) est dissociée sur le plan intramoléculaire du groupe X dans le composé représenté par la formule (I) et, de ce fait, une fluorescence dérivée du groupe X peut être émise.

2. Procédé de marquage par fluorescence d'une protéine selon la revendication 1, dans lequel l'obtention d'une protéine de fusion comprend l'obtention d'un polynucléotide codant pour la protéine de fusion, l'obtention d'un plasmide ou d'un vecteur qui peut exprimer la protéine de fusion, l'expression de la protéine de fusion dans une cellule ou l'isolement de la protéine de fusion ainsi exprimée.

3. Composé représenté par la formule générale (I) ou sel de celui-ci, 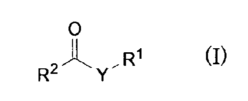 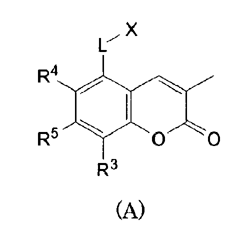 dans lequel, dans la formule (I),
Y est un atome d'oxygène ou un atome de soufre,
R¹ est un groupe alkyle inférieur, un groupe alkyle inférieur substitué par un atome d'halogène, un groupe aryle, un groupe aryle substitué par au moins un substituant choisi dans le groupe constitué d'un groupe hydroxy, d'un atome d'halogène et d'un groupe alcoxy inférieur, un groupe aralkyle inférieur ou un groupe aralkyle inférieur substitué par au moins un substituant choisi dans le groupe constitué d'un groupe hydroxy, d'un atome d'halogène et d'un groupe alcoxy inférieur, et
R² est un groupe représenté par la formule (A) comprenant la chaîne principale de coumarine,
dans lequel, dans la formule (A),
X est un groupe fluorescent,
L est un segment de liaison pour X,
R³ et R⁴ sont chacun indépendamment un atome d'hydrogène, un atome d'halogène, un groupe alkyle inférieur, un groupe hydroxy, un groupe alcoxy inférieur ou un groupe alkyle inférieur substitué par un atome d'halogène, et
R¹ est un groupe hydroxy, un groupe amino, un groupe amino qui a été monosubstitué ou disubstitué par un groupe alkyle inférieur ou un groupe alcoxy inférieur.

4. Composé ou sel de celui-ci selon la revendication 3, dans lequel
Y est un atome de soufre,
R¹ est un groupe aryle ou un groupe aryle substitué par au moins un substituant choisi dans le groupe constitué d'un groupe hydroxy, d'un atome d'halogène et d'un groupe alcoxy inférieur, et
R² est un groupe représenté par la formule (A),
dans lequel, dans la formule (A),
X est un groupe représenté par la formule (i),
L est un groupe représenté par la formule (a), la formule (b), la formule (c) ou la formule (d),
R³ et R⁴ sont chacun un atome d'hydrogène, et
R⁵ est un groupe hydroxy,
dans lequel, dans la formule (a) et dans la formule (b), n1, n2, n3, n4, n5 et n6 sont chacun indépendamment un nombre entier de 0 ou de 1 à 5 et
R¹¹ est un atome d'hydrogène ou un groupe alkyle inférieur, et,
dans la formule (c) et dans la formule (d), n1, n2, n3, n4, n5, n6 et n7 sont chacun indépendamment un nombre entier de 0 ou de 1 à 5 et
R¹¹ et R¹² sont chacun indépendamment un atome d'hydrogène ou un groupe alkyle inférieur, 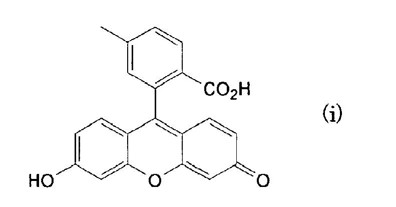 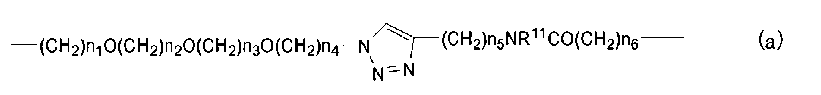 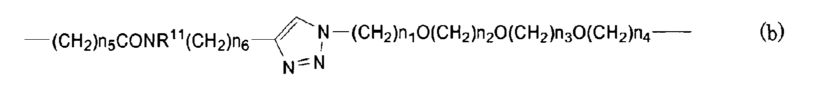
-(CH₂)n₁O(CH₂)n₂O(CH₂)n₃O(CH₂)n₄-(CH₂)n₅NR¹¹CO(CH₂)n₆NR¹²CO(CH₂)n₇ - (c)
-(CH₂)n₅CONR¹¹(CH₂)n₆CONR¹²(CH₂)n₇-(CH₂)n₁O(CH₂)n₂O(CH₂)n₃O(CH₂)n₄ - (d)

5. Composition à utiliser pour un procédé selon la revendication 1 ou la revendication 2, comprenant un composé ou un sel de celui-ci selon la revendication 3 ou la revendication 4.

6. Kit pour un procédé de marquage par fluorescence d'une protéine, comprenant une composition selon la revendication 5 et un plasmide ou un vecteur,
dans lequel le plasmide ou le vecteur est destiné au clonage d'un polynucléotide pour coder une protéine de fusion d'une protéine cible à marquer et d'une PJP ou d'une protéine dérivée d'une PJP comme définie dans la revendication 1 ou destiné à l'expression de la protéine de fusion,
le plasmide ou le vecteur comprend une séquence de bases choisie dans le groupe constitué :
1) des séquences de bases représentées par les SEQ ID N° : 18 à 29 de la liste de séquences,
2) des séquences de bases représentées par les SEQ ID N° : 18 à 29 de la liste de séquences dans laquelle les séquences de bases ont été supprimées correspondant à la suppression des 1^{er} au 24^{ème}, 25^{ème}, 26^{ème} ou 27^{ème} à l'extrémité N-terminal des séquences d'acides aminés qui sont codées avec les séquences de bases,
3) des séquences de bases dans lesquelles une à plusieurs bases des séquences de bases de 1) ou de 2) décrites ci-dessus ont été supprimées, substituées ou ajoutées, dans lequel les séquences de bases codent pour les séquences d'acides aminés dans lesquelles, lorsque la protéine de fusion se lie à la chaîne principale de coumarine dans la formule (A), la chaîne principale de coumarine dans la formule (A) est dissociée sur le plan intramoléculaire du groupe X dans le composé représenté par la formule (I), et, de ce fait, une fluorescence dérivée du groupe X peut être émise, et
4) des séquences de bases qui ont une homologie d'au moins 70 % avec les séquences de bases de 1) ou de 2) décrites ci-dessus, dans lequel les séquences de bases codent pour les séquences d'acides aminés dans lesquelles, lorsque la protéine de fusion se lie à la chaîne principale de coumarine dans la formule (A) ci-dessous, la chaîne principale de coumarine dans la formule (A) est dissociée sur le plan intramoléculaire du groupe X dans le composé représenté par la formule (I) et, de ce fait, une fluorescence dérivée du groupe X peut être émise, 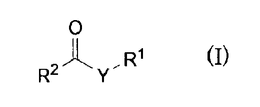 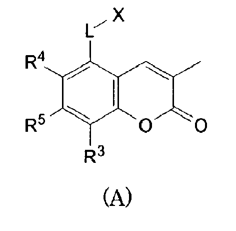
dans lequel, dans la formule (I),
Y est un atome d'oxygène ou un atome de soufre,
R¹ est un groupe alkyle inférieur, un groupe alkyle inférieur substitué par un atome d'halogène, un groupe aryle, un groupe aryle substitué par au moins un substituant choisi dans le groupe constitué d'un groupe hydroxy, d'un atome d'halogène et d'un groupe alcoxy inférieur, un groupe aralkyle inférieur ou un groupe aralkyle inférieur substitué par au moins un substituant choisi dans le groupe constitué d'un groupe hydroxy, d'un atome d'halogène et d'un groupe alcoxy inférieur, et
R² est un groupe représenté par la formule (A) comprenant la chaîne principale de coumarine,
dans lequel, dans la formule (A),
X est un groupe fluorescent,
L est un segment de liaison pour X,
R³ et R⁴ sont chacun indépendamment un atome d'hydrogène, un atome d'halogène, un groupe alkyle inférieur, un groupe hydroxy, un groupe alcoxy inférieur ou un groupe alkyle inférieur substitué par un atome d'halogène, et
R⁵ est un groupe hydroxy, un groupe amino, un groupe amino qui a été monosubstitué ou disubstitué par un groupe alkyle inférieur ou un groupe alcoxy inférieur.
